(19) Europäisches Patentamt

European Patent Office

Office européen des brevets

(11) **EP 1 334 982 A2**

(12) **EUROPEAN PATENT APPLICATION**

(43) Date of publication:
**13.08.2003 Bulletin 2003/33**

(51) Int Cl.7: **C07K 14/705**, C30B 7/00

(21) Application number: **03250762.6**

(22) Date of filing: **06.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **07.02.2002 JP 2002031571**

(71) Applicant: **National Institute of Advanced Industrial Science and Technology Tokyo 100-8901 (JP)**

(72) Inventors:
• **Ishii, N., c/o Nat.Inst. Adv. Ind. Science & Tech. Tsukuba-shi, Ibaraki 305-8566 (JP)**

• **Wang, Pi-Chao., c/o Inst. of Applied Biochemistry Tsukuba-shi, Ibaraki 305-8572 (JP)**
• **Kato, Hisamune Tsukuba-shi, Ibaraki 300-2622 (JP)**
• **Mizuno, Masashi Nagoya-shi, Aichi 466-8560 (JP)**

(74) Representative: **Maschio, Antonio D Young & Co, 21 New Fetter Lane London EC4A 1DA (GB)**

(54) **Solubilized complement receptor type 1 in crystal form**

(57) The complement receptor sCR1 which can be utilized in analyzing the three-dimensional structure of sCR1 and is very useful as a complement activity inhibitor is provided in crystal form. Animal cells producing the complement receptor sCR1 are cultured in a two-step culture process using a serum-containing medium and a serum-free medium, the culture fluid or treated culture fluid obtained is purified in a two-step process employing affinity chromatography and size exclusion chromatography, and the purified product is crystallized.

**Description**

BACKGROUND OF THE INVENTION

1. FIELD OF THE INVENTION

The present invention relates to crystals of soluble-form complement receptor type 1 (sCR1), a complement activity inhibitor in which such crystals are used, a method of producing such crystals, a method of cultivating animal cells for obtaining such crystals, and a method of purifying them.

2. DESCRIPTION OF THE PRIOR ART

Complement receptor 1(CR1), which is a membrane glycoprotein, exists on the membranes of erythrocytes, monocytes/macrophages, granulocytes, B cells, some T cells, spleen follicular dendritic cells, and glomerular podocytes. CR1 binds C3b and C4b, hence is called C3b/C4b receptor. Its most amino acid sequence has already been determined (Science, 249: 146.151 (1990)).

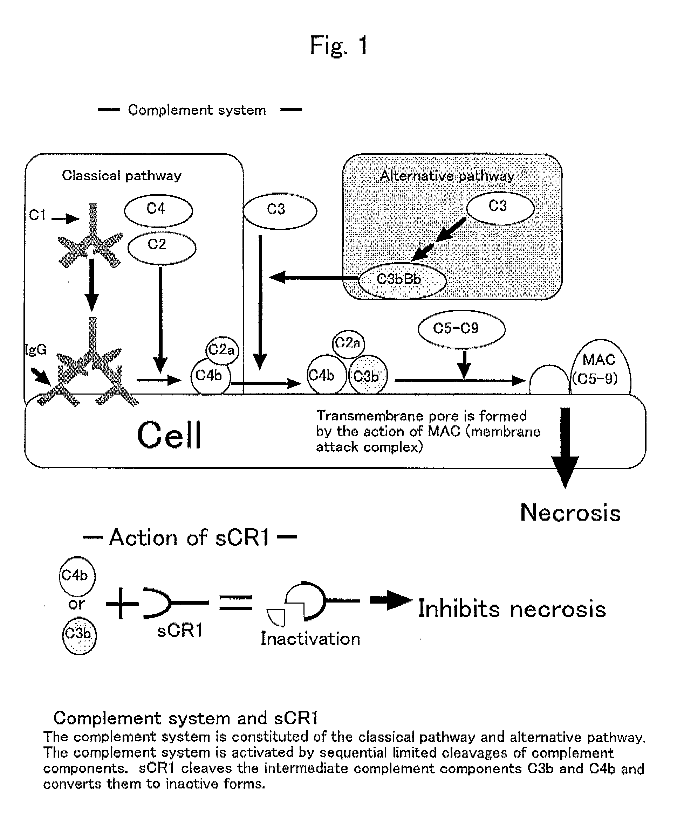

sCR1 is also known as a substance inhibiting the activation of the complement system; it inhibits the classical pathway and alternative pathway of the complement system. As a background of this pathway inhibition, it is known that the complement components C1-C9 repeatedly undergo restricted cleavage in the complement pathways. The sCR1 binds activated C3b and C4b, which are intermediates in the complement system, and inactivates and destructs these complement components by its cofactor activity in cooperation with factor I, which is a complement factor, to thereby eventually prevent cell death (necrosis) (Fig. 1).

Furthermore, sCR1 is known to inhibit oxidative nucleophil burst, complement-mediated hemolysis, and the *in vitro* production of C3a and C5a, which are complement components. Reportedly, it exhibits in vitro activity also in reverse passive Arthus reaction (reaction similar to allergic reaction), in suppressed postischemic myocarditis, and in necrosis. Therefore, sCR1 is an important regulatory protein in the complement system and functions to prevent autolytic host tissue destruction by complement activation.

As regards the prior art of cell supernatant recovery methods and complement receptor sCR1 purification methods, the following publications may be cited: J. Immunol., vol. 146, No. 1, 250-256 (1991); Eur. J. Immunol., vol. 26, No. 8, 1729-1735 (1996); and J. of Biol. Chem., vol. 274, No. 16, 11237-11244 (1999). In all of the works reported, ATCC CRL-10052 CHO cells were used and sCR1 was purified from the cell culture supernatant. However, these techniques require the use of a number of expensive cell proliferation apparatus and column systems for purification and involve complicated steps, so that the purification efficiency is decreased and sCR1 is mostly denatured. Further, since the whole cell culture supernatant contains a various kinds of contaminants as well, the purity is considered to be low. Protein concentration seems to have been succeeded, namely the protein concentration in each sample after concentration is high, but the proportion of sCR1 contained therein is low; the degree of concentration thus cannot be considered to be high.

It is of course difficult to crystallize such less purified grades of sCR1. Any method has not yet been found out for realizing the crystallization of a soluble-form complement receptor type 1 (sCR1) which has a molecular weight of not lower than 200 kDa, and for elucidating the steric structure of the same based thereon, in spite of earnest desire therefor. While methods have so far been developed for crystallizing various proteins, no crystals have been obtained of membrane proteins of a molecular weight of not lower than 200 kDa, in particular of complement receptor type 1 (sCR1) occurring in various reticuloendothelial system cell membranes, since such proteins are difficult to retain their respective intact states under cell culture and purification conditions.

In view of such problems in the prior art, it is an object of the present invention to develop means for obtaining crystals of the complement receptor sCR1 and thus provide the complement receptor sCR1 in a purified form and in a crystal form, which can be used for steric structure analysis of the complement receptor and is very useful as a complement activity inhibitor.

SUMMARY OF THE INVENTION

As a result of intensive investigations made to accomplish the above object, the present inventors have newly developed a two-step cell culture process comprising cell proliferation culture using a serum-containing medium and cell culture using a serum-free medium in which the production of sCR1 by cells is improved, and a rapid two-step column purification process comprising two type of column chromatography, namely affinity column chromatography and size exclusion chromatography. Further, it was found that when these means are used, sCR1 can be produced in an intact state. Thus, the inventors have succeeded for the first time in crystallizing complement receptor type 1 (sCR1).

Thus, the present invention is concerned with the following (1) to (21):

(1) A crystal of the soluble-form complement receptor type 1 (sCR1) in crystal form;

(2) A crystal of the soluble-form complement receptor type 1 (sCR1) as described above under (1), obtained by crystallization of the soluble-form complement receptor type 1 purified by a process comprising the following steps a and b:

a: propagating animal cells introduced with a gene for soluble-form complement receptor type 1 (sCR1) in a serum-containing medium and then cultivating the cells in a serum-free medium for causing them to produce soluble-form complement receptor type 1 (sCR1); and

b: step of purifying the thus-produced soluble-form complement receptor type 1 (sCR1) by the successive use of an affinity column chromatography and a size exclusion column chromatography;

(3) A crystal of soluble-form complement receptor type 1 (sCR1) as described above under (1) or (2) which contains no transmembrane domain;

(4) A crystal of soluble-form complement receptor type 1 (sCR1) as described above under any of (1) to (3) wherein it has no glyco-chain;

(5) A crystal of soluble-form complement receptor type 1 (sCR1) as described above under any of (1) to (4), obtained by the process mentioned above in which the purification step(s) a and/or b are(is) carried out in the presence of a surfactant;

(6) A crystal of soluble-form complement receptor type 1 (sCR1) as described above under any of (1) to (5), wherein the crystallization means comprises a combination of a precipitation procedure using a protein precipitating agent and a vapor diffusion method;

(7) A complement activity inhibitor which comprises a crystal of the soluble-form complement receptor type 1 (sCR1) according to any of (1) to (6);

(8) A method of producing a crystal of soluble-form complement receptor type 1 (sCR1) which comprises crystallizing the soluble-form complement receptor type 1 (sCR1) obtained by a process comprising the following steps a and b:

a: propagating animal cells introduced with a gene for the soluble-form complement receptor type 1 (sCR1) gene in a serum-containing medium and then cultivating the cells in a serum-free medium for causing them to produce the soluble-form complement receptor type 1 (sCR1); and

b: purifying the produced soluble-form complement receptor type 1 (sCR1) by the successive use of an affinity column chromatography and a size exclusion column chromatography;

(9) A method of producing a crystal of the soluble-form complement receptor type 1 (sCR1) as described above under (8), wherein the gene for the soluble-form complement receptor type 1 (sCR1) is one resulting from mutation or deletion in the transmembrane domain-encoding region;

(10) A method of producing a crystal of soluble-form complement receptor type 1 (sCR1) as described above under (9), wherein the soluble-form complement receptor type 1 (sCR1) is excreted extracellularly;

(11) A method of producing a crystal of soluble-form complement receptor type 1 (sCR1) as described above under any of (8) to (10), wherein the cultivation step(s) a and/or b are(is) carried out in the presence of tunicamycin;

(12) A method of producing a soluble-form complement receptor type 1 (sCR1) as described above under any of (8) to (11) wherein the purification step(s) a and/or b are(is) carried out in a state wherein the soluble-form complement receptor type 1 (sCR1) is dispersed by means of a surfactant;

(13) A method of producing a crystal of soluble-form complement receptor type 1 (sCR1) as described above under any of (8) to (12) wherein the crystallization means comprises a combination of a precipitation procedure using a protein precipitating agent and a vapor diffusion method;

(14) A two-step cell culture method, comprising propagating animal cells introduced with a gene for soluble-form complement receptor type 1 (sCR1) in a serum-containing medium, followed by cultivation in a serum-free medium;

(15) A two-step culture method as described above under (14) wherein the soluble-form complement receptor type 1 (sCR1) gene is one resulting from mutation or deletion in the transmembrane domain-encoding region;

(16) A two-step culture method as described above under (15) wherein the animal cells introduced with the soluble-form complement receptor type 1 (sCR1) gene extracellularly secrete the soluble-form complement receptor type 1 (sCR1);

(17) A two-step culture method as described above under any of (14) to (16) wherein the cultivation step(s) is (are) carried out in the presence of tunicamycin;

(18) A two-step process for purifying a soluble-form complement receptor type 1 (sCR1), which comprises purifying the soluble-form complement receptor type 1-containing animal cell culture medium treated or untreated, obtained by the two-step culture method according to any of (14) to (17), by using an affinity column chromatography and a size exclusion column chromatography successively;

(19) A two-step purification process as described above under (18) wherein the animal cell culture supernatant containing the soluble-form complement receptor type 1 (sCR1) is directly applied to an affinity column chromatography;

(20) A two-step purification process as described above under (18) or (19), wherein the soluble-form complement receptor type 1 (sCR1) is subjected, in a dispersed state by a surfactant, to purification by using an affinity column chromatography and/or a size exclusion column chromatography; and

(21) A complement activity inhibitor wherein the soluble-form complement receptor type 1 (sCR1) obtained by the two-step purification process described above under any of (18) to (20) is used.

BRIEF DESCRIPTION OF THE DRAWINGS

Fig. 1 is a schematic illustration of the mechanism of the complement system and the action of sCR1.

Fig. 2 is a schematic illustration of the process for crystallizing the soluble-form complement receptor type 1 (sCR1) according to the invention.

Fig. 3 is a photomicrograph of the state of each culture fluid in two-step cell culture.

Fig. 4 is a representation of the electrophoretograms of the culture fluids recovered by the conventional cell culture method and the novel two-step cell culture method.

Fig. 5 is a schematic illustration of the two-step cell culture method in which sCR1 is subjected to glyco-chain elimination.

Fig. 6 is a photomicrograph of states of cells in media during the two-step cell culture method using tunicamycin.

Fig. 7 is a representation of electrophoretograms of fractions eluted in purifying sCR1 by means of a heparin column.

Fig. 8 is an electrophoretogram of a fraction eluted in gel filtration column chromatography.

Fig. 9 is a representation of electrophoretograms of fractions eluted in purifying Aglyco-sCR1 using a heparin column.

Fig. 10 is an electrophoretogram of a fraction eluted in purifying Aglyco-sCR1 using a heparin column.

Fig. 11 is a schematic illustration of anti-sCR1-binding antibody binding sites of anti-sCR1 antibodies and sCR1.

Fig, 12 is a representation of the results of western blotting, showing that four antibodies have specificity to sCR1.

Fig. 13 is a representation of the results of western blotting, showing that four antibodies have specificity to Aglyco-sCR1.

Fig. 14 is a schematic illustration of the method of crystal preparation by sitting drop vapor diffusion.

Fig, 15 is a stereoscopic photomicrograph of crystals of sCR1.

Fig. 16 is a photograph showing the results of observation of sCR1 under an electron microscope.

Fig. 17 is a photograph showing the results of observation of Aglyco-sCR1 under an electron microscope.

Fig. 18A is a schematic illustration of the restricted cleavage of C3b by Aglyco-sCR1 and factor I, and Fig. 18B is an electrophoretogram showing the results of an experiment on such cleavage.

Fig. 19 is a schematic illustration of the restricted cleavage of C3b by Aglyco-sCR1 and factor I, with an electrophoretogram showing the results of an experiment on such cleavage.

Fig. 20 is a graphic representation of the relationship between sCR1 concentration and CH50.

Fig. 21 is a graphic representation of the relationship between sCR1 concentration and ACH50.

Fig. 22 is a graphic representation of the relationship between the amount of sCR1 added and iC3b concentration.

Fig. 23 is a graphic representation of the relationship between the amount of sCR1 added and SC5b-9b concentration.

Fig. 24 is a photographic representation of the results of a cytotoxic experiment using mouse cranial bone-derived stroma cells.

Fig. 25 is a schematic illustration of the procedure for an animal experiment using LPS-sensitized 512 shock model rats.

DETAILED DESCRIPTION OF THE INVENTION

In the following, the present invention is described in more detail.

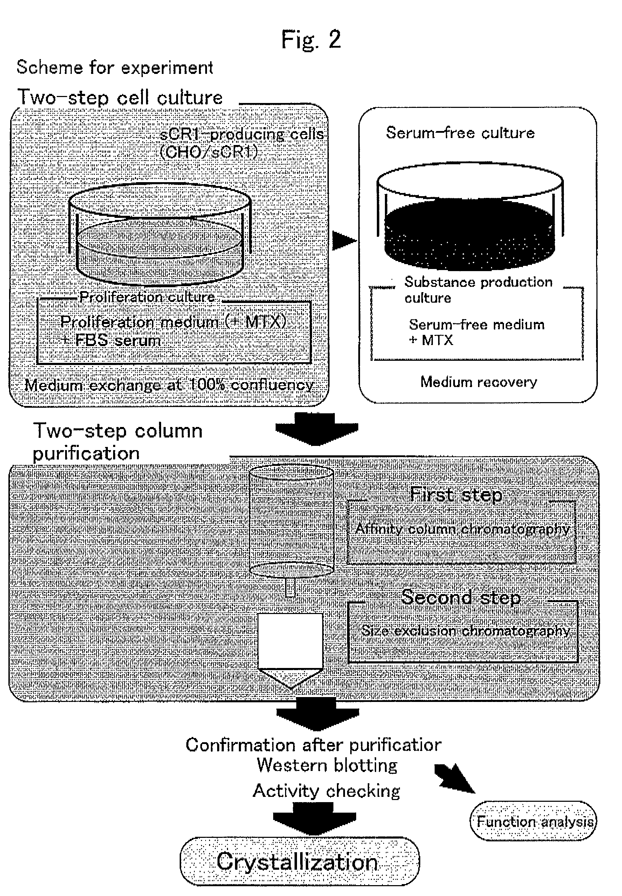

The present inventors have developed a two-step cell culture method comprising cell propagation culture in a serum-containing medium and culture in a serum-free medium for improving the production of the sCR1 protein as well and a two-step column method for protein purification comprising affinity column purification and a size exclusion column chromatography. They found that when these means are used, soluble-form complement receptor type 1 (sCR1) can be produced in an intact state (capable of retaining the complete structure thereof) and, accordingly, crystals of soluble-form complement receptor type 1 (cSR1) can be produced with ease. The process of preparing crystals of soluble-form complement receptor type 1 (sCR1) is outlined in Fig. 2.

The means for preparing crystals of soluble-form complement receptor type 1 (sCR1) according to the invention makes it possible to prepare crystals of sCR1, sCR1 is a high molecular weight protein, and thus its steric has not yet been analyzed. Such crystallization can provide not only information on the steric structure and functions of sCR1 but also a very pure and highly safe complement activity inhibitor with extremely high purity and high safety.

The success in crystallizing soluble-form complement receptor type 1 (sCR1) in the present invention depends on the fact that the protein can be obtained with good efficiency and in high purity and high concentration by means of the two-step cell culture method and two-step column purification method.

The sCR1 to be used in the practice of the invention is one resulting from mutation, deletion or like modification of the transmembrane site of CR1 on the gene thereof. It comprises a water-soluble portion with a molecular weight of about 220 kDa. This soluble CR1 fragment shows physiological activity, i.e. can bind active forms of C3b and/or C4b, which are intermediates in the complement system.

On the other hand, sCR1 is known as a substance inhibiting the activation of the complement system, as mentioned above; it inhibits the classical pathway and alternative pathway of the complement system. As a background of this pathway inhibition, it is known that the complement components C1-C9 repeatedly undergo restricted cleavage in the complement pathways. Then sCR1 binds activated C3b and C4b, which are intermediates in the complement system, and destructs and inactivates these complement components by exerting its cofactor activity in cooperation with factor I, a complement factor, to thereby eventually prevent cell death (necrosis).

Furthermore, sCR1 is known to inhibit *in vitro* oxidative nucleophil burst, complement-mediated hemolysis, and the production of C3a and C5a, which are complement components. Reportedly, it exhibits *in vitro* activity also in reverse passive Arthus reaction (reaction similar to allergic reaction), in suppressed postischemic myocarditis, and in necrosis. Therefore, sCR1 is an important regulatory protein in the complement system and functions to prevent autolytic destruction of the host tissue by complement activation.

The sCR1 according to the invention is a product of expression by CHO cells introduced with a human tonsil-derived sCR1 gene. More specifically, it is a product of expression by Chinese hamster ovary (CHO) cells containing the plasmid pBSCR1c/pTCSgpt, which has been deposited with the ATCC with an accession number of CRL10052. Since, as mentioned above, CR1 molecules substantially free of any transmembrane domain can be secreted out of cells, it can be recovered from the cell culture supernatant.

In crystallizing the complement receptor sCR1 according to the invention, in order to address the problems of the prior art cell supernatant recovery and complement receptor sCR1 purification methods (J. Immunol., vol. 146, No. 1, 250-256 (1991); Eur. J. Immunol., vol. 26, No. 8, 1729-1735 (1996); and J. of Biol. Chem., vol. 274, No. 16, 11237-11244 (1999)), the process for crystallizing the complement receptor sCR1 including the step of cell culture have been reviewed, and fundamental improvements have been made. Thus, a two-step culture method and a two-step column purification method have newly been developed, making it possible to rapidly purify the sCR1 protein at high purity and with high efficiency.

In the two-step culture according to the invention, the above-mentioned cells secrete sCR1 with high efficiency. Fundamentally, the rate of proliferation of animal cells can be accelerated by addition of serum, such as FBS (fetal bovine serum), and various amino acids. Therefore, the first step cell proliferation culture is carried out in a serum-containing medium. However, the presence of serum increases the content of contaminants or impurities, making the protein purification procedure complicated. Therefore, in the second step, a serum-free medium is used so that the number and amounts of contaminant species may be markedly reduced to facilitate the purification of the sCR1 protein. This purification method requires no expensive column, and is simple and easy to perform at low cost.

In the two-step column purification following cell culture, sCR1 can be purified with a high purity and a high concentration by two steps of column chromatography only in two steps while all complicated purification steps have been omitted. In the first step, an affinity column chromatography showing high affinity to sCR1 is used to accomplish purification of sCR1 with a higher level of purity. In the second step, sCR1 can be separated from other proteins owing to the difference in molecular size.

The purified sCR1 was subjected to SDS-PAGE and western blotting, among others, for confirmation of the eluate fraction and of the intact sCR1 structure and, then, investigations were made about the conditions of crystallization by the vapor diffusion method. Further, the purified sCR1 was checked for its cofactor activity and *in vitro* and *in vivo* activities.

[Method of crystallizing sCR1]

The method of crystallization according to the invention is now described more specifically in the following.

(1) Two-step cell culture process

i) In accordance with the present invention, sCR1 is obtained by cell proliferation culture (step 1) in a serum-containing medium using ATCC CRL10052 Chinese hamster ovary (CHO) cells introduced with a human tonsil-derived sCR1 gene, for instance, followed by serum-free culture (step 2) in a serum-free medium for producing sCR1 alone.

The medium to be used in the above proliferation culture (step 1) is, for example, α-MEM (Gibco BRL), F-12 (sigma), DMEM/F-12 (Sigma), F-10 (Sigma), or RPMI (Sigma) containing FBS (fetal bovine serum, Sigma) and 500 nM methotrexate (MTX, Sigma), the culture is carried out for 0 to 336 hours.

The serum-free medium to be used in the serum-free culture (step 2) is, for example, a medium (e.g. ASF104 (Ajinomoto), α-MEM (Gibco BRL), F-12 (Sigma), DMEM/F-12 (Sigma), F-10 (Sigma)) containing transferrin and/or insulin and addition of 500 nM methotrexate (MTX; Sigma). The culture is carried out for 0 to 360 hours. Characteristically, the cell culture is always carried out at 37°C in the presence of 5% $CO_2$.

ii) The sCR1 in the serum-free culture supernatant obtained in the above manner can be purified in the subsequent two-step column purification process. This process can give several tens of milligrams, per liter, of a protein with a 90% or higher purity. In the practice of the invention, the scale can be increased, hence the scale is not restricted.

In the practice of the invention, glyco-chain elimination may be made using tunicamycin (Wako) in the cell culture system. Generally, a protein which is added by glyco-chains in a proportion of 20% or more, is difficult to form a crystal with good quality, because it has a regular three-dimensional arrangement due to the flexibility thereof after purification. Therefore, the glyco-chain removal by tunicamycin influences on the crystal core formation. Since sCR1 is a glyco-chain-containing protein, it is desirable that the effects of the glyco-chain be taken into consideration. However, glyco-chain-containing sCR1 can also be crystallized in accordance with the present invention.

This glyco-chain elimination can be realized by providing a tunicamycin invasion period by adding tunicamycin during cell culture, whereby a product (Aglyco-sCR1) resulting from N-linked glyco-chain elimination from sCR1 is obtained. The tunicamycin invasion period is provided by adding tunicamycin, to a concentration of 2-10 μg/ml, to the culture after cell proliferation in the cell proliferation culture step. It lasts 12 to 48 hours. As for the culture conditions on that occasion, the serum-containing medium used in the proliferation culture is used, and MTX is also added. After invasion, the medium is exchanged for the serum-free medium for use in the substance production culture step, and the Aglyco-sCR1 production is allowed to proceed for 12 to 60 hours. Thus, it becomes possible to obtain glyco-chain-deprived sCR1 alone.

(2) Two-step column purification process

i) sCR1 can be purified from the cell culture supernatant containing sCR1 secreted by the two-step column purification process. In this purification process, an affinity column chromatography is used in the first step, and a a size exclusion column chromatography in the second step.

The two-step column purification method according to the invention has overcome the problems encountered in the prior art, such as mentioned just below. The problems are, among others, elution of a denatured protein due to changes in pH, complicated exchange of buffers used in an ion exchange column, for instance, protein denaturation due to ammonium sulfate precipitation, and procedural conditions incapable of retaining the intact sate of sCR1. Therefore, in the first step, a gradient of a salt such as NaCl (Wako) is employed for the elution from the affinity column, and in the second step, the intact protein is separated and eluted depending on the molecular size thereof through size exclusion chromatography.

ii) In the first step, the affinity chromatography is carried out using heparin, for instance. Heparin-Sepharose CL-6B (Pharmacia Biotech, Code No. 17-0467-01), for instance, may be used as the heparin-binding carrier. Heparin is a glucosaminoglycan having, as a skeleton, a structure of repetitions of a disaccharide composed of a uronic acid residue derived either from D-glucuronic acid or from L-iduronic acid and D-glucosamine. It is not uniform in molecular weight, and the molecular weight ranges from 3,000 to 100,000. Reportedly, heparin binds tyrosine kinase type receptors, which are heparin-binding growth factors (HBGFs), von Willebrand factor (vWF), which has an effective action in hemostasis, and various cytokines. It is also reported that heparin and sCR1 have strong affinity for each other (Shigeharu Nagasawa: Men-eki to Seitai Bogyo (Immunity and Living Body Protection) (vol. 10)).

As for the elution conditions in affinity chromatography, elution is carried out using a buffer solution containing a surfactant low in micelle forming rate. For protein elution, the eluent buffer also contains 0.1 to 2 M NaCl. The surfactant concentration is adjusted within the range of 0.01% to 0.5% (w/v) relative to the whole volume of the buffer solution. At the same time, the pH is adjusted so that it may fall within the range of from slight acidity to the alkaline side (pH 6.0-10.0). Thus, the buffer is preferably selected so that its pH may fall within the above range. In the second step, namely in size exclusion chromatography, the technique of gel filtration chromatography, for instance, is employed. By this, separation is effected based on the sizes of molecules and, substantially, a form of molecular sieve is provided. Since it is desired that any interaction should not take place between the matrix and solute, a matrix substance which is generally inactive is preferred. It is also desirable that the matrix be hard and very porous. While gels are relatively soft, gels having increased hardness have been developed and, in the practice of the invention, a HPLC gel filtration column chromatography (Tosoh's analytical column TSKgel G3000SWXL), on which separation can be maintained even at high flow rates, is preferably used. In the size exclusion chromatography in this second step, a surfactant with lower micelle forming rate than that used in the first step, while the eluent buffer in this second step does not contain any salt. The eluent flow rate is 2-5 ml/min in the first step of purification, and that in the second step is 0.5-1 ml/min.

The surfactant to be used in the first step is, for example, CHAPS or the like, and that to be used in the second step includes, among others, DDM (n-dodecyl β-D-maltoside) and DM (n-decyl-β-D-maltopyranoside).

iii) After each of the first step and second step of purification, a conventional concentration procedure using a pressure concentrator (Amicon), an ultrafiltration membrane (Amicon) or the like is repeated. Normally, the above-mentioned concentrator is used, but the apparatus to be used is not limited to such concentrator. The pressure concentrator is operated normally under a pressure of 2-3 kPa, and the centrifugation on the ultrafiltration membrane-containing concentrator is carried out at 2000-3000 x g.

(3) Confirmation or detection of sCR1 in the two-step cell culture process and two-step column purification process

i) After application of the above-mentioned two-step column purification process, electrophoresis can be carried out using a 7.5% polyacrylamide gel containing 0.1% SDS to determine the yield of sCR1, the contaminant content, the eluate fractions containing sCR1 and the efficiency of purification. As for the SDS-PAGE conditions, the electrophoresis is always carried out under a reducing condition (in a state in which the cysteine residues in the protein are cleaved from each other) using DDT (dithiothreitol (Sigma)). After electrophoresis, the protein is subjected to CBB (Bio-Rad) staining and/or silver staining (Daiichi Pure Chemicals), which is higher in detection sensitivity than CBB staining, for detecting the band of the protein.

ii) For checking whether the protein purified is sCR1 or not and whether the structure thereof is retained or not, western blotting is carried out. In western blotting, electrophoresis is carried out on a 7.5% polyacrylamide gel containing 0.1% SDS, followed by transfer onto a nitrocellulose membrane (0.45 μm; Amersham Pharmacia Biotech). After transfer, blocking is effected using 5% skimmed milk (Snow Brand Milk Products). Mouse anti-sCR1 mAb is used as the primary antibody, and sheep anti-mouse horseradish peroxidase (HRP)-lined IgG mAb (Amersham Life Science) as the secondary antibody. After three times of washing operations, the ECL reagent (Western blotting detection reagent; Amersham Life Science) is added for the detection of the secondary antibody.

(4) Step of crystallizing sCR1

In accordance with the present invention, a satisfactory protein concentration can be obtained by an ordinary concentration procedure, such as the above-mentioned ultrafiltration. Then, using the thus-concentrated sCR1 solution, sCR1 is crystallized. Usable as the crystallization technique are, for example, the batch method which comprises adding a precipitating agent to the protein solution to a state of supersaturation and allowing the mixture to stand, the liquid-liquid diffusion method (free interface diffusion method) which comprises pouring a precipitating agent into a thin tube or capillary to the middle, gently forming a protein interface thereon and allowing the solvent or precipitating agent to migrate through that interface, the dialysis method which comprises using a capillary or a dialysis device called button, covering the outside thereof with a dialyzing membrane and immersing the same in a precipitating agent solution to thereby gradually lower the solubility, and the vapor diffusion method which utilizes the phenomenon that a volatile solvent or precipitating agent migrates by diffusion through the gaseous phase. Preferred among them is the vapor diffusion method by which the crystal growth can be observed and investigations using various precipitating agents can be made with ease.

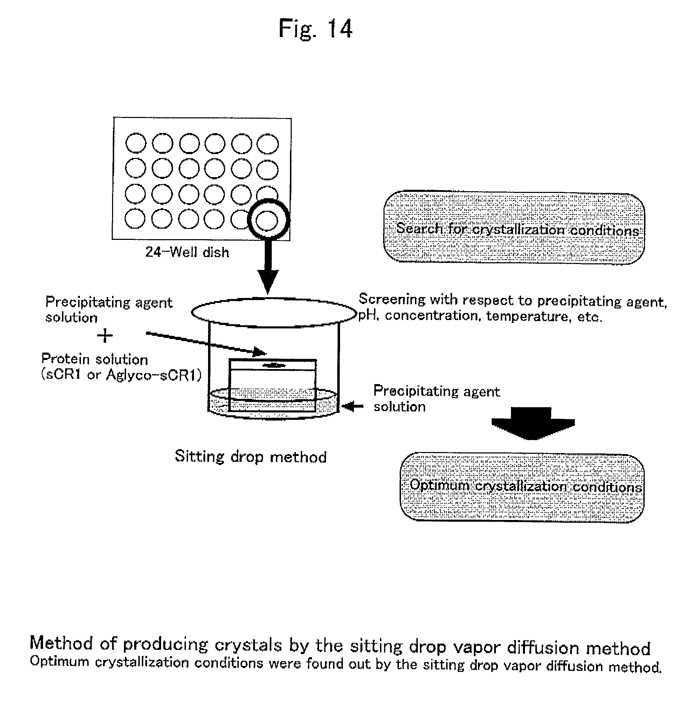

The vapor diffusion method utilizes the mechanisms of an equilibrium being attained by vapor diffusion, through the gaseous phase, from a liquid drop (of a mixture of a protein solution and a small amount of a precipitating agent) to the precipitating agent solution or by a reverse process. The method is characterized by its utilizing the phenomenon that vapor diffusion between the protein solution and precipitating agent solution causes increases in both protein concentration and precipitating agent concentration to allow crystallization to occur in the supersaturated region. The vapor diffusion method includes the hanging drop technique and sitting drop technique according to the form of mounting. The sitting drop method is preferred, however. The reason is as follows. Since sCR1 resulting from genetic modification of the corresponding membrane protein is not readily soluble in water or buffer solutions, a surfactant is used in the purification system. The sample after protein purification thus contains the surfactant and is weak in surface tension. The hanging drop technique may possibly allow the drop to spread on the cover glass. The crystal preparation method using the sitting drop technique is schematically shown in Fig. 14.

Crystals can be obtained by mixing the precipitating agent (buffer, surfactant, organic solvent) to be used for crystallization by the vapor diffusion method with the protein solution in a ratio of 1:1.

On that occasion, the sCR1 crystal preparation conditions (e.g. buffer, surfactant, organic solvent, pH, temperature) and the method of crystallization (e.g. vapor diffusion method, batch method, free interface diffusion method) are not particularly restricted.

(5) Observation of sCR1 under an electron microscope

In accordance with the present invention, the thus-obtained crystals of sCR1 can be used in collecting diffraction data using X rays or synchrotron radiation, namely in crystal structure analysis, as well as in three-dimensional structure analysis by electron microscopy. Electron microscopy has been used in case of difficulty in preparing a large crystal or for obtaining rough information for crystallization. However, owing to the recent remarkable advances in observation techniques by electron microscopy and in peripheral tools, it is now possible to construct a three-dimensional model from two-dimensional crystal data. Therefore, the present inventors first attempted to apply electron microscopy by ice embedment, which enables high resolution analysis, to unimolecular observation of the sCR1 protein obtained. For investigating optimal conditions for crystallization in the practice of the invention and for obtaining a general view of sCR1, observations and analyses were performed using transmission electron microscopy following negative staining with a heavy metal.

(6) Techniques for confirming the functions of sCR1 Confirmation of in vitro activities

1) In view of the function, sCR1 obtained in accordance with the invention is characterized by its retaining ability to function as a cofactor of complement factor I (factor I) and as a decay accelerating factor. These physiological activities can be measured by carrying out *in vitro* assays.

That sCR1 obtained in accordance with the invention has complement regulating functions has been confirmed by a cofactor activity experiment using purified C3 and factor I and by a decay accelerating activity experiment using IgM-sensitized sheep erythrocytes (CH50) and rabbit erythrocytes (ACH50). Based on the results of the cofactor activity or decay accelerating activity experiment, it has been established that sCR1 can inactivate the C3/C5 converting enzyme and can restrictedly cleave C3b/C4b and thus retains the functional characteristics of CR1.

2) The extent of inhibition, by sCR1, of complement system activation via the classical pathway and alternative pathway can be quantitatively measured by *in vitro* assaying by ELISA using the supernatant of CH50.

3) Whether sCR1 obtained in accordance with the invention can inhibit the complement system was checked using animal cells in a cytotoxic experiment using non inactivated human serum (human serum retaining complement activity). As mentioned hereinabove, human serum contains complement components and activation of the complement system causes cell death (necrosis). Therefore, by carrying out an in vitro cytotoxic experiment, it is possible to observe, under a microscope, the extent of inhibition of the complement system.

4) As mentioned above, the cofactor activity for confirming the activity of sCR1 involves a reaction utilizing the restricted cleavage of C3b in cooperation with factor I. Since C3b is an unstable substance, C3ma (methylamine-treated human C3), which is stable, was used as a substitute. C3ma as the substrate of cofactor activity, factor I and sCR1 were mixed together, and the reaction was allowed to proceed at 37°C for 8 hours. After reaction, the sample was subjected to 7.5% gel SDS-PAGE (under a reducing condition), followed by CBB (Bio-Rad) staining for band detection.

5) CH50 and ACH50 values were determined by the microplate method using 96-well plates (Iwaki). The microplate method comprises diluting and altering the volume of the sample on a plate, adding erythrocytes thereto and allowing the reaction to proceed, and subjecting the microplate as it is to centrifugation and absorbance measurement. In these CH50 and ACH50 determinations, positive detection is obtained only when all the complement components, C3, B, D, P and C5-C9, are present. The 50% hemolysis method is used for the assays, and the results are given in terms of CH50/ml and ACH50/ml, respectively. The buffers used were GVB (gelatin veronal buffer) for CH50, and GGVB (glucose gelatin veronal buffer) and EGTA-Mg$^{2+}$-GGVB (GGVB containing 0.1 M EGTA and 0.02 M Mg solution) for ACH50. As for the reaction conditions, erythrocytes, NHS and buffer were mixed up on a microplate using a multichannel pipetter for microplates and, after 60 minutes of incubation at 37°C, the plate was centrifuged at room temperature and at 1600-2000 x g for 10 minutes and, then, absorbance measurements were carried out using a microplate reader adjusted to the wavelengths 415/630 nm.

The actual observation values were derived from the absorbance values using a statistics application software, Microsoft Excel and used for graphic representation.

6) ELISA was carried out using Quidel's kit and according to the manufacturer's protocol. This kit includes an anti-complement component antibody-bound 96-well microplate, an HRP (horseradish peroxidase)-bound anti-complement component antibody, and a substrate for luminescence. The assay was carried out by the sandwich technique (by which the target protein is sandwiched between two antibodies and the actual observation value can be detected as a more accurate value). Every reaction was carried out at room temperature, and each absorbance value was read at the wavelength 405 nm. Each protein concentration was determined according to a calibration curve (constructed in advance using known concentrations of the protein) and used for graphic representation.

7) In the cytotoxic experiment for observing the *in vitro* activity of sCR1, a mouse cranial bone-derived stroma cell line (PA-6) was used as animal cells. Non-inactivated human serum (NHS) was used as the serum. Mouse PA-6 cells were sowed on 24-well plates 1-2 days prior to the day of experiment and cultured until confluency. After arriving at confluency, the medium was added, inactivated NHS (heated inactivated pooled NHS; 56°C, 40 minutes) and an sCR1 solution prepared by using NHS and phosphate-buffered saline (PBS) were added, and incubation was carried out in the presence of 5% $CO_2$ at 37°C for 60 minutes, followed by morphologic observation.

Confirmation of an *in vivo* sCR1 activity

For evaluating the in vivo therapeutic activity of sCR1, the acute lethal shock reaction (which is a reaction similar to allergic reaction and known as anaphylactic shock) was utilized. This is an acute inflammatory response caused by the interaction between an antigen in a tissue and a circulating antibody. This is used as a classical example of an *in vivo* inflammatory response and is characterized by immune complex formation, complement activation, inflammatory cell recruitment, edema, and tissue injury (P. Baily and A. Sturm, 1983, Biochem. Pham. 32:475). From the experimental viewpoint, it is a general practice to establish an animal model of the reverse passive Arthus reaction by intravenous injection of an antigen, followed by administration of an antibody. In evaluating sCR1 according to the invention for its *in* vivo therapeutic efficacy, however, nude model rats (rats in which no immune system functions; model animals in which the complement reaction can be induced by administration of lipopolysaccharide (LPS); LPS-sensitized 512 shock model) administered with a monoclonal antibody (mAb against rat Crry,512) capable of specifically binding to the complement regulating protein occurring on the cell membrane were used.

The following examples further illustrate the present invention. They are, however, by no means limitative of the scope of the invention.

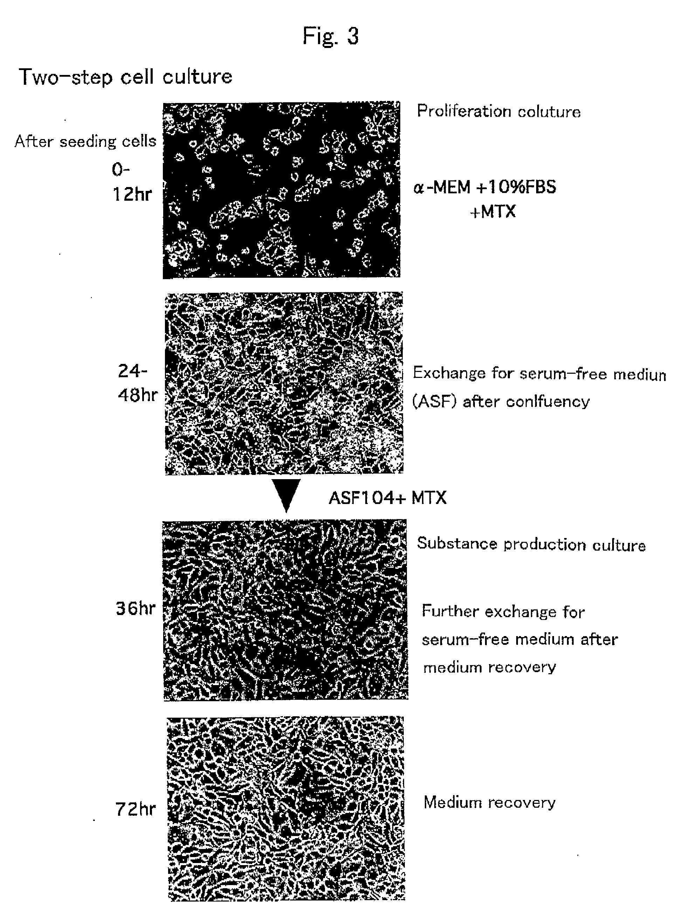

Example 1 Two-step cell culture (Fig. 3)

(1) Cell proliferation culture (first step)

CHO cells (ATCC CRL-10052) expressing sCR1 were cultured according to the protocol recommended by the ATCC. Thus, cells were cultured and proliferated using Alpha modified Eagle medium (α-MEM, Gibco BRL) supplemented with 10% fetal bovine serum (PBS; Gibco BRL), essential amino acids, L-glutamine, proline, penicillin (100 U/ml) -streptomycin (100 μg/ml), and 500 nM methotrexate and using a 150 mm dish (capacity: 25 ml). Since, on that occasion, the medium was free of sodium hydrogen carbonate ($NaHCO_3$), 2.2 g, per liter, thereof was added for pH adjustment. The medium should be free of deoxyribonucleotides and ribonucleotides so that it may adapt itself to CHO cells. After arrival of the cell density at about 70-80% confluency, cell were subcultured in new 150 mm dishes. For the subculture, the 25-ml container was washed with two or three portions of phosphate-buffered saline (PBS; Nissui) and then cells were detached from the plate with trypsin solution(0.25% trypsin-1 mM EDTA-4Na, Gibco BRL) and put into the 25-ml containers at the concentration of about 2.0 x $10^6$ cells/ml. The medium used was α-MEM (proliferation medium) supplemented with 10% fetal bovine serum (FBS) and penicillin-streptomycin. This operation was repeated until about 50 plates were obtained. Cells were always cultured in an incubator in the presence of 5% $CO_2$ at 37°C.

(2) Substance production culture (serum-free culture) (second step)

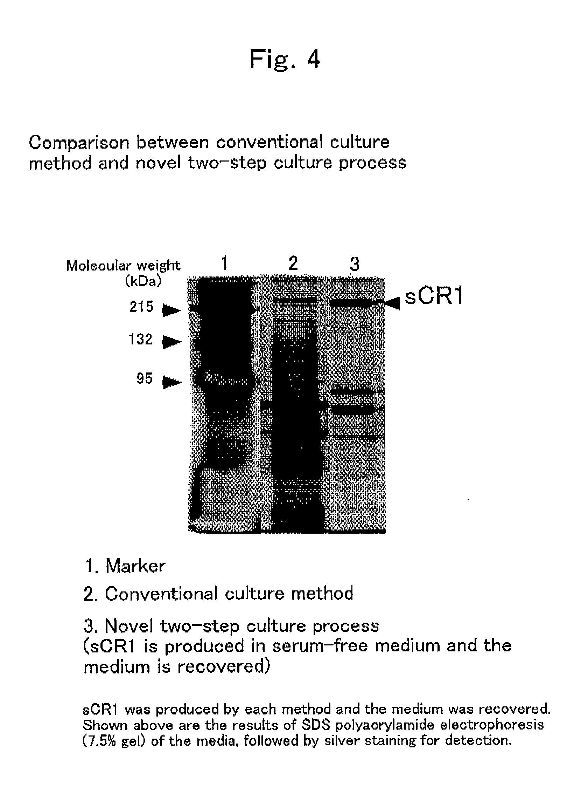

Then, when the CHO cell density arrived at about 80-90% confluency on the proliferation medium, the medium was replaced with a substance production medium (serum-free medium). The medium used was a serum-free medium (Ajinomoto; ASF104; albumin-free). Reportedly, the ASF104 medium makes it possible to attain high levels of antibody productivity with various hybridomas. The medium was prepared by blending the principal components, buffer and additives of this medium. After medium preparation, methotrexate, which was used in the proliferation medium, was added, and the resulting medium was designated as ASF (+) (+: methotrexate-containing). Prior to addition of this medium, the 150 mm dishes confluent with cells were washed two or three times with phosphate-buffered saline (PBS), and then the ASF(+) medium prepared was added. Thereafter, cultivation was carried out at 37°C for 36 hours using a 5% $CO_2$ incubator. After the 36 hours, the sCR1-containing medium was recovered and, in exchange thereof, the ASF(+) medium was added and cultivation was again carried out at 37°C for 36 hours (total cultivation time: 72 hours) in a 5% $CO_2$ incubator. The sCR1-containing medium recovered was centrifuged at 800-1,000 x g for 5 minutes using a sting rotor (Tomy) to remove contaminants. The supernatant alone was collected by decantation. Then, the supernatant recovered was filtered through a 0.45 μm pore size filter (Millipore), and the filtrate was used as a solution for purifying sCR1. For checking the yield of sCR1 and the amount of impurities, each filtrate fraction was subjected to 7.5% polyacrylamide SDS gel electrophoresis under a reducing condition. The results are shown in Fig. 4. It is evident that the amount of other components (contaminants) than sCR1 is small in the two-step cell culture shown in this example.

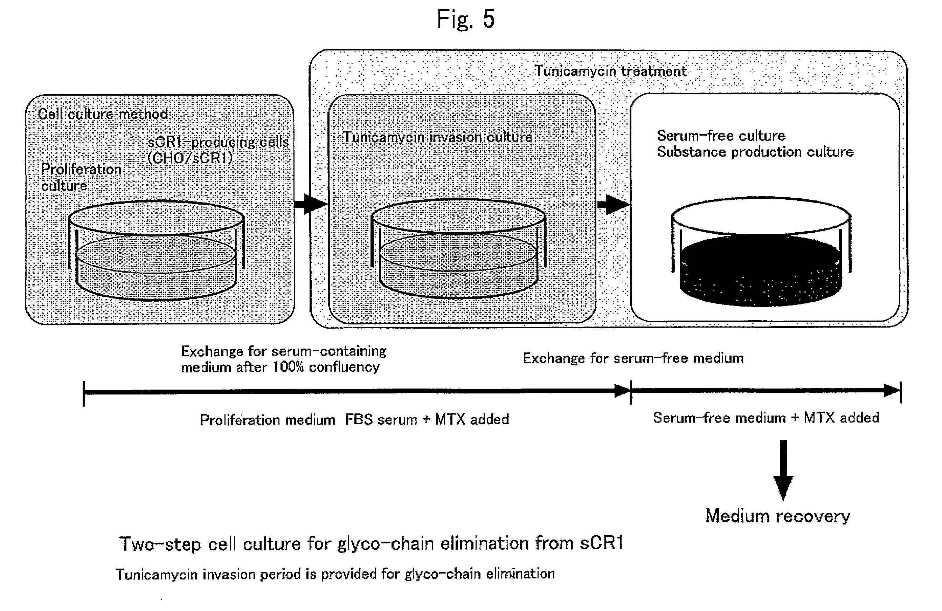

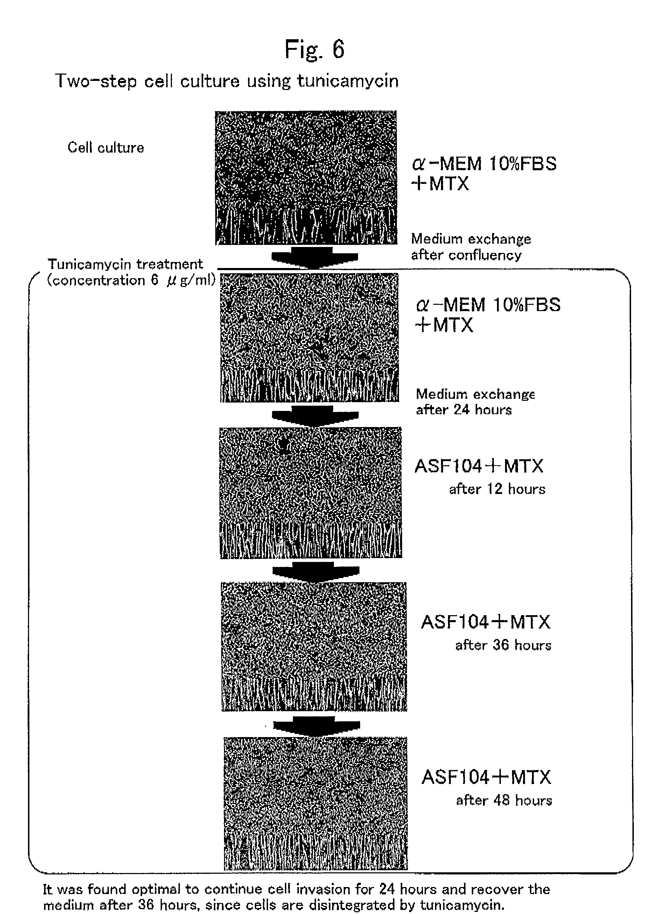

(3) Method of sCR1 (Aglyco-sCR1) resulting from glyco-chain elimination with tunicamycin (Figs. 5 and 6)

Tunicamycin (Wako) invasion culture was carried out after the proliferation culture. First, tunicamycin solution was prepared at a concentration of 200 μg/ml using phosphate-buffered saline (PBS; Nissui). Since tunicamycin is not soluble in the solution, an organic solvent such as an aprotic polar solvent, for example dimethyl sulfoxide (DMSO; Wako) or isopropanol (2-propanol; Wako), was added in an amount of 0.2-0.4% relative to the whole amount. Then, the CHO cells at about 80-90% confluency in proliferation medium in 150 mm dishes (Greiner) were washed two or three times of phosphate-buffered saline (PBS). Thereto was added the medium ($\alpha$-MEM (Gibco BRL), containing 10% FBS), which was used in the proliferation culture, and the tunicamycin preparation prepared in advance was added to the whole amount in each dish to give a concentration of 6-8 μg/ml. After addition, the tunicamycin invasion culture was carried out at 37°C for 24-36 hours (optimally 24 hours to avoid cell disintegration) in a 5% $CO_2$ incubator. After invasion culture, each dish was again washed two or three times, and the serum-free medium (ASF(+)) was added. On this occasion, too, tunicamycin was added to the whole amount in each dish at a concentration of 6-8 μg/ml (optimally 6 μg/ml). Substance (Aglyco-sCR1) production culture in the presence of tunicamycin was again performed at 37°C for 36 hours in a 5% $CO_2$ incubator. After the 36 hours, the medium was recovered and treated in the same manner as mentioned above. The filtrate obtained was used as a solution for purifying Aglyco-sCR1.

Example 2 Process for purifying sCR1 from the culture supernatant

(1) Method of purifying sCR1 using an affinity carrier

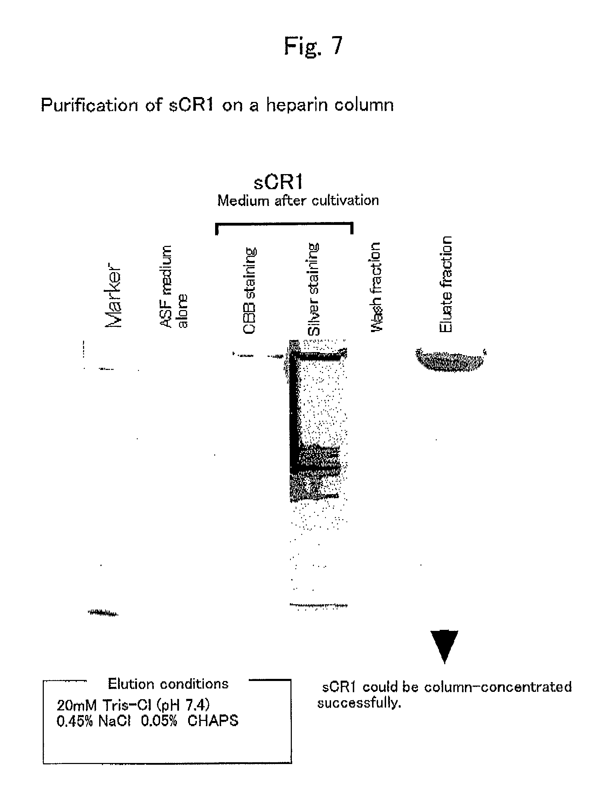

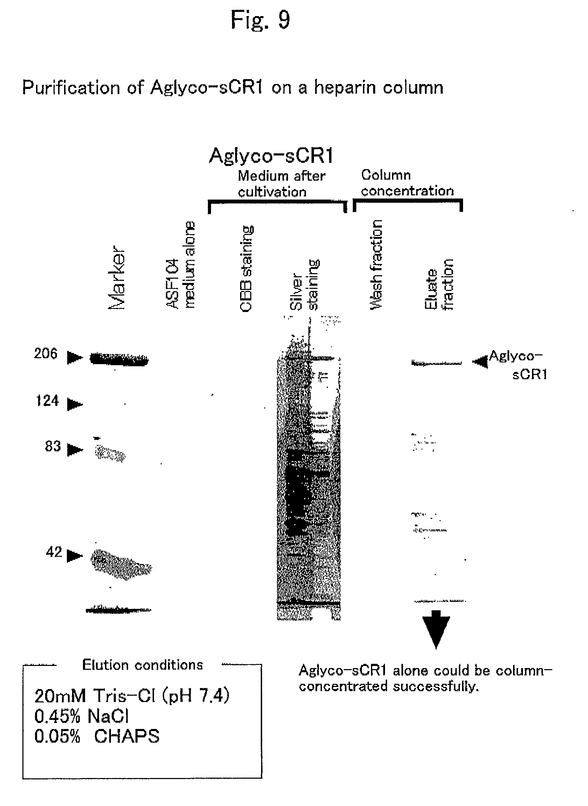

Laboratory scale purification of sCR1 was accomplished by heparin affinity chromatography. The heparin carrier used was Pharmacia Biotech's Heparin-Sepharose CL-6B (Code No. 17-0467-01). The heparin column was prepared according to the protocol recommended by the manufacturer. The carrier was allowed to swell in 200 ml, per gram of the carrier, of ultrapure water for 15 minutes and, then, the gel was poured slowly into a column (diameter 1.5 x 15 cm) . (Each gram gave about 4 ml of swollen carrier, and about 10-15 ml of the swollen carrier was used.) Then, the swollen carrier placed in the column was washed with at least 10 volumes of ultrapure water, and the column was equilibrated by further passing 5 volumes, relative to the carrier volume, of 20 mM Tris-Cl buffer (pH 7.4) containing 0.05% of CHAPS (ICN) (each time, the flow rate was 5 ml/min). Then, the sCR1-containing medium (about 1 L) was directly passed through the column to cause the carrier to trap sCR1 (sCR1) in the supernatant (flow rate: 5 ml/min). All the effluent fractions passing through the column were recovered. Then, the buffer solutions containing 0.15 M, 0.45 M, 0.75 M and 1 M NaCl (Wako), respectively, were applied, each in an amount of 60 ml, to the column in that order, and 10-ml eluate fractions were collected (flow rate: 3 ml/min). After elution, 0.1 M Tris-Cl buffer (pH 8.5) containing 5% NaCl and 0.1 M sodium acetate buffer (pH 5.0) containing 0.5% NaCl were passed through the column alternately three times each in a volume at least three times of the column volume and, then, at least 5-fold volumes of the column volume, of the equilibrating buffer was passed through the column to thereby remove contaminants in the column and regenerate the column (each time the flow rate was 5 ml/min). From the next run and thereon, the column operation was started from the passage of the equilibrating buffer, and the same procedure was repeated.
Thereafter, for checking which fraction(s) contained sCR1 and for checking the yield and impurity content, each eluate fraction was subjected to 7.5% polyacrylamide SDS gel electrophoresis under a reducing condition. After electrophoresis, Coomassie Brilliant Blue (CBB; Bio-Rad) staining was carried out. As a result, a single protein band was detected at a position (about 220 kDa) exceeding about 200 kDa (in the case of sCR1). This is the size of the sCR1 protein as expected from the estimated amino acid sequence thereof. In the case of sCR1 with no glyco-chain added (Aglyco-sCR1), a single protein species was detected at a position of about 190 kDa, just below about 200 kDa. This is supposedly due to a decrease in molecular weight because of no glyco-chain added. These results are shown in Fig. 7 and Fig. 9.
Then, the eluate fractions obtained (with high purity) were pooled and 10 to 15-fold concentrated in a pressure concentrator (cutoff molecular weight: 100 kDa; Amicon). For further concentration, the sCR1 concentrate after concentration to about 10 ml was again concentrated using an ultrafiltration membrane (Centri-plus; cutoff molecular weight 100 kDa; Millipore) until the volume was reduced to about half.

(2) Method of purification by size exclusion chromatography

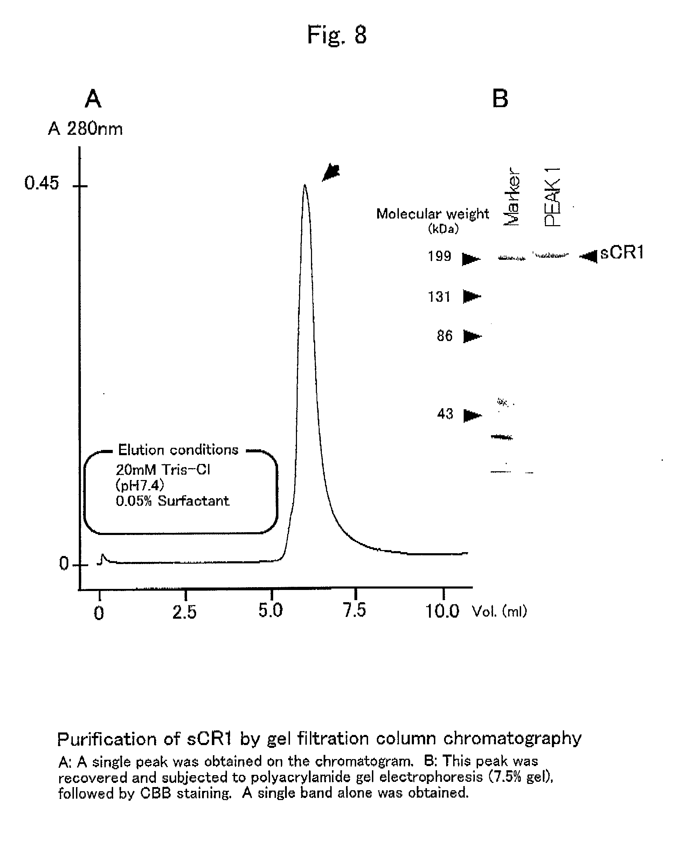

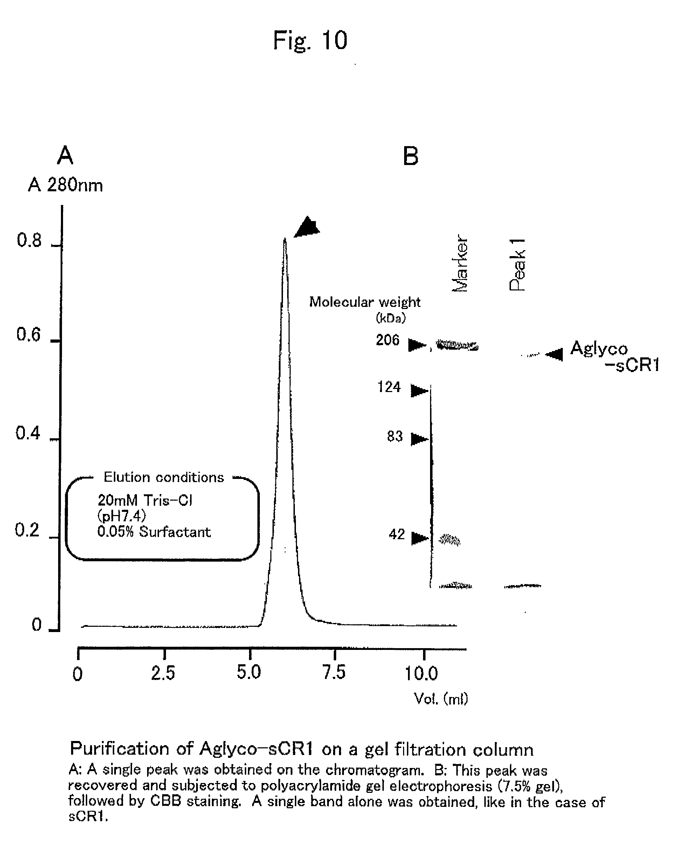

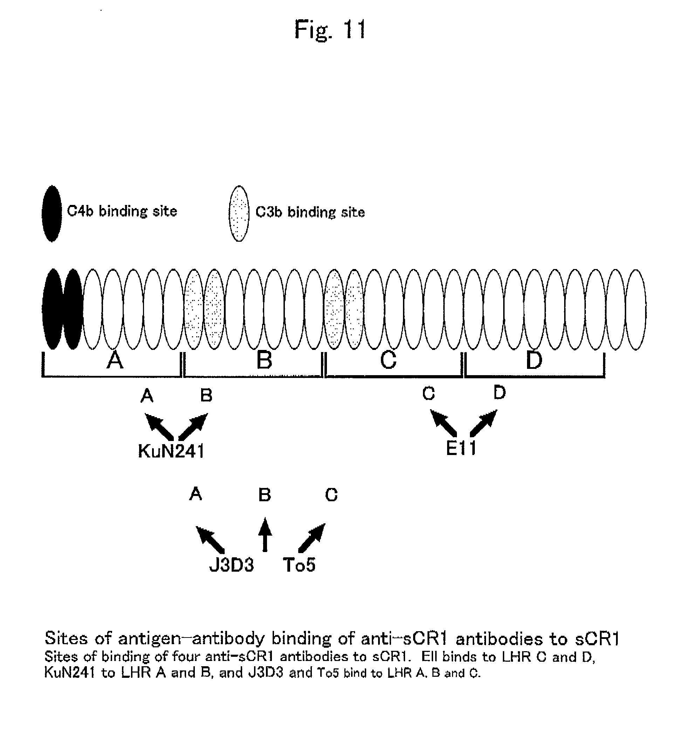

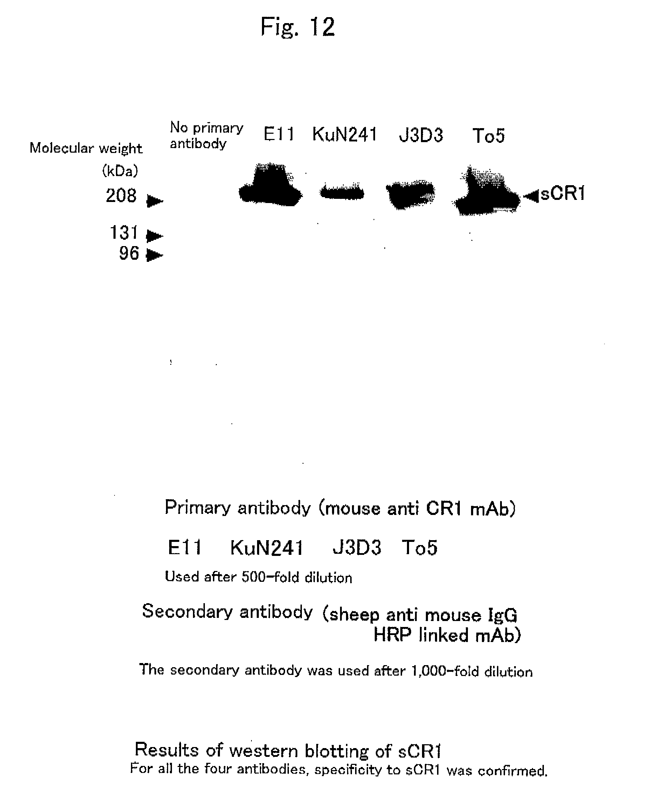

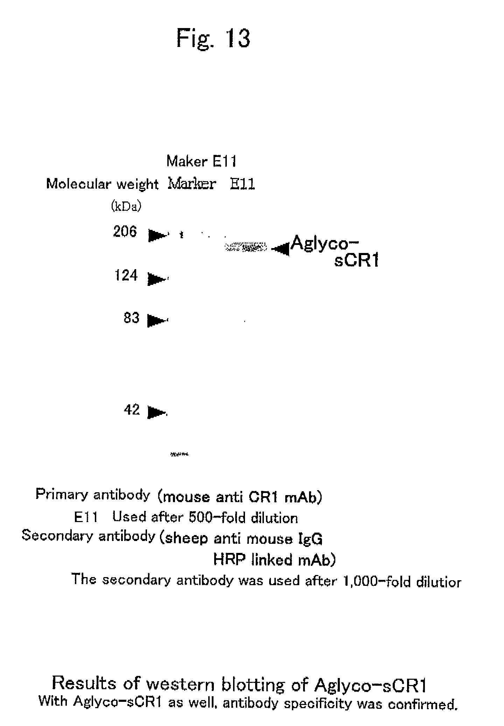

In this example, HPLC gel filtration column chromatography (Tosoh's analytical column TSKgel G3000SWXL), which ensures separation even at high flow rates, was employed. Although 20 mM Tris-Cl buffer (pH 7.4) containing 0.05% CHAPS (ICN) was initially used as the elution buffer, CHAPS, which is a surfactant, is not very suitable for crystallization of the soluble form of membrane protein-derived sCR1. Therefore, an eluent was prepared by dissolving DDN (n-decyl-beta-D-maltopyranoside; ICN) having a sugar moiety added to a concentration of 0.05% relative to the whole amount of the eluent. After injection of 500 µl of the concentration sCR1 solution mentioned above into the column, one eluate peak was obtained after elution with about 6-7 ml (Fig. 8), and about 1-2 ml of the peak fraction was recovered. This procedure was repeated several times, and about 10 ml of such eluate was recovered.
Then, for checking the impurity content or the absence of any impurity, each eluate fraction was subjected to a 7.5% polyacrylamide SDS gel electrophoresis under a reducing condition, followed by CBB staining for band detection (Fig. 8). As a result, it was confirmed that the eluate obtained had high purity (for both of sCR1 and sugar moiety-free Aglyco-sCR1 (Fig. 8 and Fig. 10)). For checking whether the protein actually obtained was actually sCR1 or not and whether the structure of the protein was damaged or not during the purification process, western blotting was carried out under a reducing condition using four primary antibodies (E11 (Pharmingen), J3D3 (Beckman Coulter), To5 (Dako) and KuN241 (Neo Makers), which are anti-sCR1 mAb's) and anti-mouse Ig mAb (Amersham Life Science) as a secondary antibody. The sites of binding of the four anti-sCR1 mAb's to sCR1 are schematically shown in Fig. 11. It is reported that the antibodies used in this example specifically bind to sCR1 at LHR C and D (in the case of E11), LHR A and B (in the case of KuN241), and LHR A, B and C (in the case of J3D3 and To5) (Clin. Exp. Immunol. 1998, 112: 27-33). Based on these results indicating such specificity for each antibody, it was confirmed that the protein obtained was sCR1 and that the structure thereof was retained (Fig. 12). With Aglyco-sCR1, however, only E11 was labeled to a slight extent (Fig. 13) and, therefore, it is supposed that the antibodies recognize the glyco-chains as well. Paradoxically, it is considered that the glyco-chain elimination by tunicamycin had been achieved completely.
Thereafter, for further concentration of the sample after purification by gel filtration chromatography to give a sample for crystallization, the sample was concentrated using an ultrafiltration membrane (Amicon). On the other hand, for preparing a sample for use in *in vitro* and *in vivo* biochemical assays, the sample was dialyzed using a dialysis membrane (Spectra/Por, cutoff molecular weight 12-14000),
**[0055]** It was finally confirmed that sCR1 obtained by the two-step cell culture process and two-step purification process according to the invention can be recovered in a yield of 10-20 mg per liter of the medium recovered (in the case of sCR1) or 2-3 mg per liter of the medium recovered (in the case of Aglyco-sCR1).

Example 3 Method of crystallizing sCR1

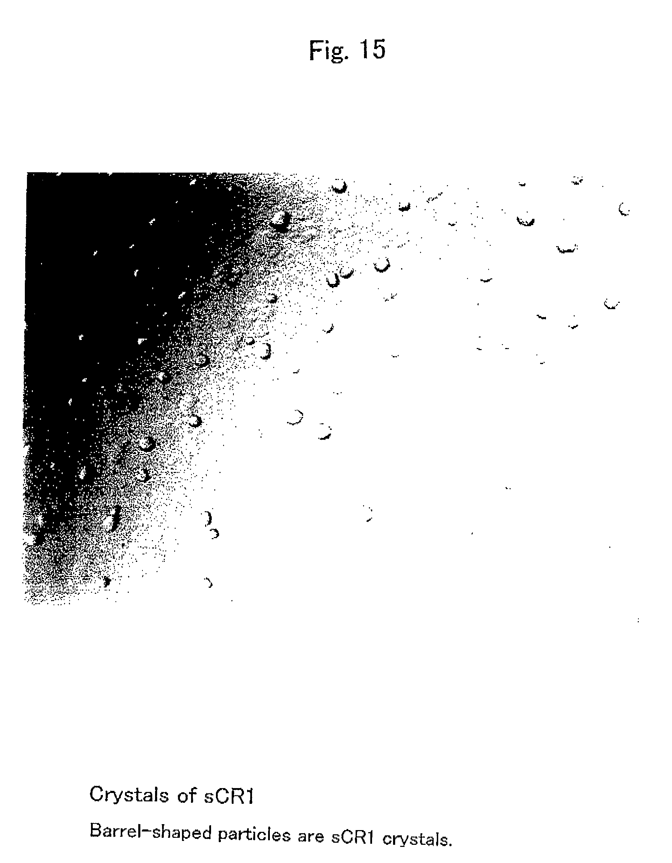

An experiment in crystallizing sCR1 after a sufficient concentration was obtained was carried out by the sitting drop vapor diffusion method. The method of the experiment was as follows. First, a 24-well dish (Iwaki) was provided and fine dust was removed from each well by air spraying. Abridge for the sitting drop technique was placed in each well, and grease was applied to the mouth portion of each well. Then, 500 µl of a mother liquor (containing a precipitating agent) to be subjected to vapor diffusion was poured into each well below the bridge. First step conditions searching was carried out by screening using Hampton Research's crystal screening kit. After addition of the mother liquor, 2 µl of the purified sample was added to the cavity of each bridge, and 2 µl of the mother liquor as taken from the well was added, followed by thorough pipetting. Then, a glass-made cover was placed on the mouth of the well and pressed from above by means of tweezers to create a tightly closed state. All the 24 wells were treated in the same manner, followed by 2 to 8 weeks of standing at 25°C, whereby crystals could be obtained (Fig. 15). As for the optimum crystallization conditions on that occasion, (1) a mother liquor composition comprising 28% PEG-400, 0.1 M Na Hepes pH 7.5, and 0.2 M calcium chloride and (2) a mother liquor composition comprising 30% PEG-400, 0.1 M Na Hepes pH 7.5, 0.2 M magnesium chloride gave good results. In particular, the former composition (1) gave good crystals. Thereafter, based on the above-mentioned optimum crystallization conditions, the crystallization experiment was again carried out for reproducibility checking using a mother liquor composition comprising 25-35% PEG-400, 0.1 M Na Hepes pH 7.5, and 0.2 M $CaCl_2/MgCl_2$. In each case, similar crystals could be obtained.

Example 4 Observation of sCR1 under an electron microscope

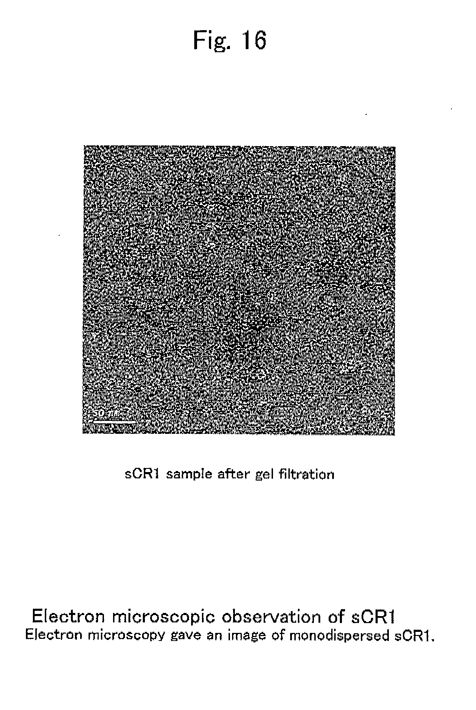

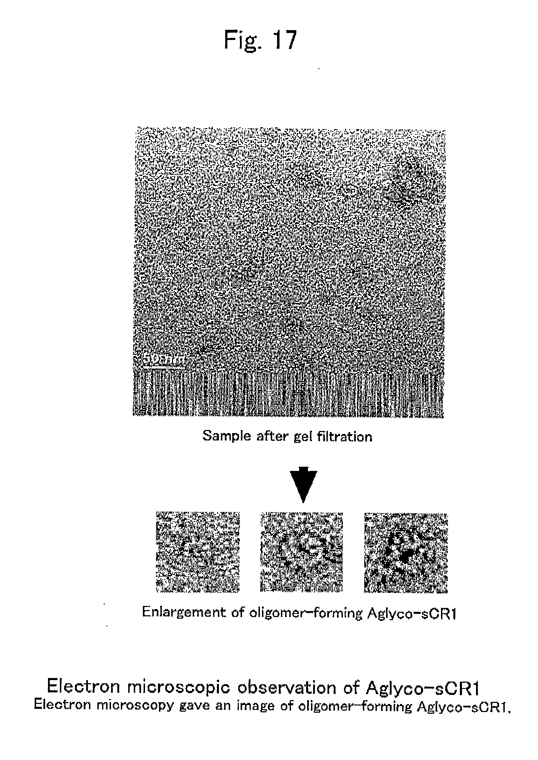

The purified grade of sCR1 as obtained by HPLC gel filtration column chromatography in Example 2 (2) was observed under an electron microscope. In the electron microscopic observation, the protein (sCR1) was placed on a hydrophilic support membrane, followed by negative staining. The experimental procedure was as follows. A grid coated with a vapor deposited carbon layer was provided, and the carbon was activated by utilizing glow discharge, for instance, under vacuum to thereby make the carbon hydrophilic. Then, 5 µl of the sCR1 sample (200-400 µg/ml) was placed thereon and, after waiting for 20-30 seconds, the sample was soaked up using a filter paper moistened in advance, a staining agent (1% uranyl acetate) was placed thereon. After the passage of a time (20-30 seconds) adequate for adsorption, the agent was again soaked up with a filter paper. The grid carrying the sample was placed on a grid holder and inserted into the inside of the electron microscope tube and, after focusing and other adjustments, the observation was carried out. As a result, an image showing the dispersion of sCR1 in a unimolecular manner was obtained (Fig. 16). For Aglyco-sCR1, an image indicating oligomer formation was obtained (Fig. 17).

Example 5 Confirmation of *in vitro* activities of sCR1

The following experiments were carried out using the purified grades of sCR1 and Aglyco-sCR1 as obtained by HPLC gel filtration column chromatography in Example 2 (2).

(1) Confirmation of the cofactor activity

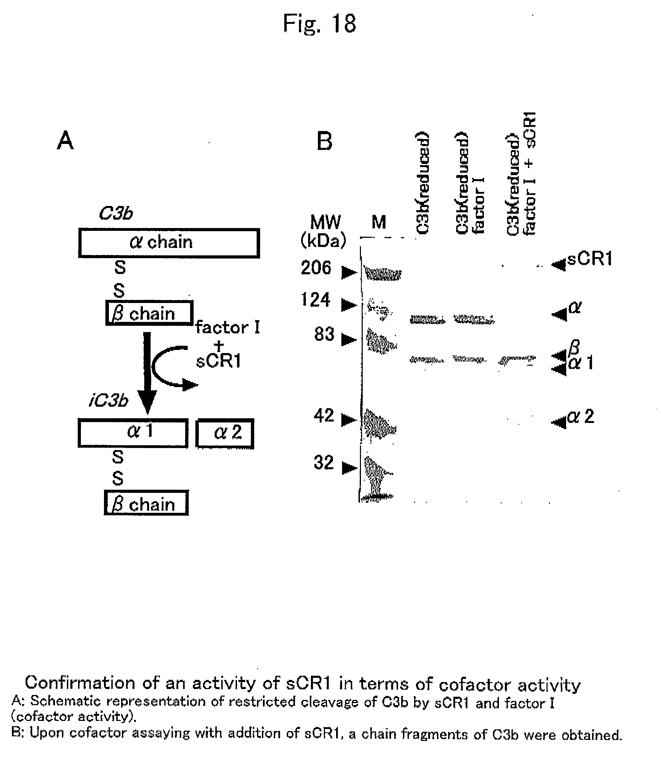

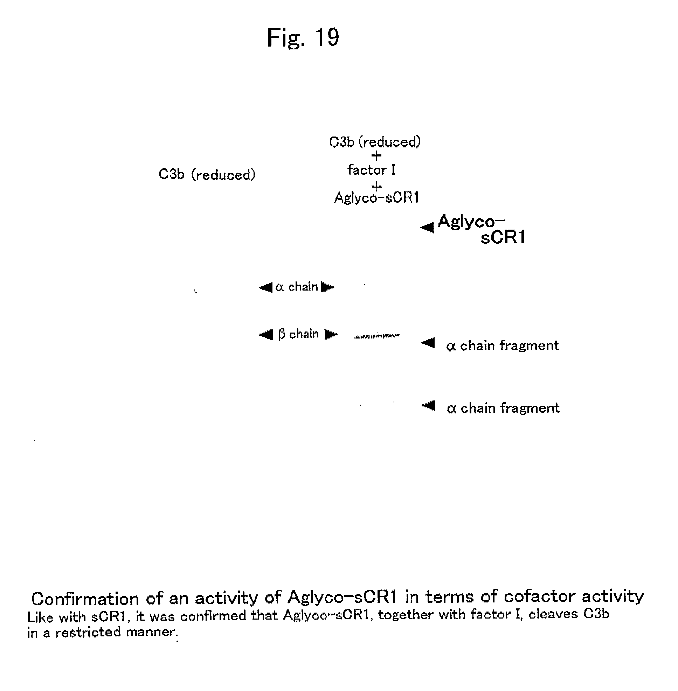

sCR1 was checked for its activity in terms of cofactor activity utilizing the restricted cleavage of C3b in cooperation with factor I.
since C3b is an unstable substance, C3ma (methylamine-treated human C3), which is stable, was used as a substitute. C3ma (20 μl, 20 μg) as the substrate of cofactor activity, 40 μl (0.5 μg) of factor I and 40 μl (6 μg) of sCR1 were mixed together, and the reaction was allowed to proceed at 37°C for 8 hours. After reaction, 20-μl portions of the sample were subjected to 7.5% gel SDS-PAGE (under a reducing condition), followed by CBB staining for band detection. sCR1 obtained in accordance with the invention cleaved the α chain of C3ma restrictedly owing to its cofactor activity in cooperation with factor I (Fig. 18). This result suggested that sCR1 purified retained its activity. Similar results were obtained with Aglyco-sCR1 as well (Fig. 19).

(2) Decay accelerating activity measurement

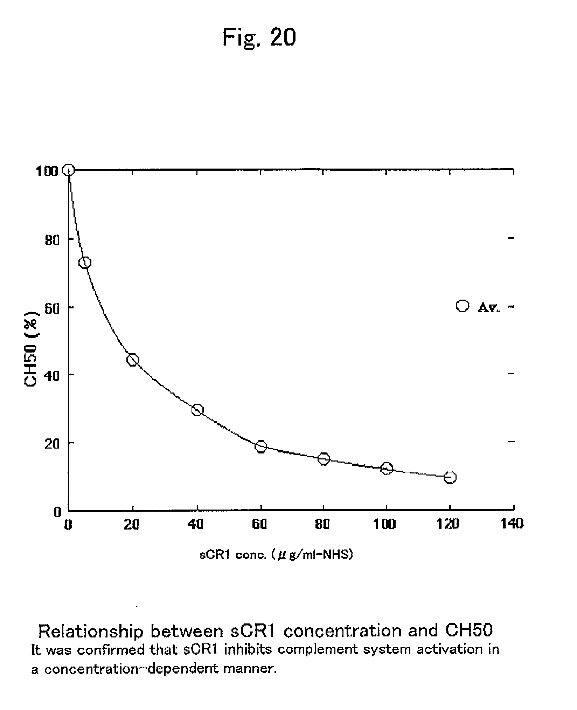

i) The name CH50 (complement hemolysis 50) is derived from the unit quantity for causing 50% hemolysis, hence originally a name of unit. However, it is often used as a synonym for complement value. The measurement was carried out as follows. A specified number of EAs (erythrocytes sensitized with an antibody (with the antibody adhering thereto) (E = erythrocyte, A = antibody)) were mixed with varied amounts of the sample, the reaction was allowed to proceed at 37°C for 1 hour, each mixture was then centrifuged, and the hemoglobin concentration in each supernatant was measured using a spectrophotometer. The hemolysis percentages were thus determined. The complement amount necessary for causing hemolysis of 50% of EAs added is taken as one CH50 unit, and the complement amount per unit volume of the original sample is expressed in CH50 units. Thus, the unit is CH50/ml. In this example, the microplate method using 96-well plates was employed. The microplate method comprises diluting and changing the volume of the sample on a plate, adding EAs thereto and allowing the reaction to proceed, and subjecting the microplate as it is to centrifugation and absorbance measurement. For CH50 determinations, EAs (sensitized sheep erythrocytes (with a rabbit antibody adhering thereto)), GVB (gelatin veronal buffer), NHS (normal human serum) and a series of dilutions were prepared in advance. The experiments were carried out according to the protocol for the microplate method (Shigeharu Nagasawa: Seitai to Men-eki Bogyo I, published 1997). First 5VB (stock veronal buffer; Na barbital 5.095 g, NaCl 41.5 g. 1 N HCl 18.5 ml, distilled water to make the total amount 1,000 ml) was prepared and, then, GVB (gelatin 1.0 g, 5VB 200 ml, 0.15 M Ca and 1 M Mg solution 1 ml, total amount 1,000 ml), 0.9% NaCl, sCR1 and NHS were prepared.
Then, a 1.5 x $10^8$ cells/ml EA preparation and 1-120 μg/ml-NHS (ml) preparations (prepared by preparing a dilution series of sCR1, followed by 10-fold dilution with NHS) were prepared. GVB and distilled water were applied to a 96-well plate except for the uppermost row, and sCR1-NHS solutions prepared in the same manner by preparing a dilution series followed by further 10-fold dilution with GVB were applied in 75-μl portions to the vacant uppermost row. Using a diluter, a 50-μl portion of the contents in each uppermost row well was transferred to the next row well therebelow and this type of transfer was repeated toward each lowermost row well to thereby produce a continuous series of 25-μl 3/2-fold dilutions. GVB (50 μl) was added to each well. Two or three wells (control wells) showing complete hemolysis or no hemolysis were prepared, and 75 μl of GVB and 75 μl of distilled water were added to each well. Then, 25 μl of the EA preparation prepared was added to each well, the reaction was allowed to proceed at 37°C for 60 minutes and, after reaction, the microplate was subjected to 10 minutes of centrifugation at 1,600-2,000 x g. After centrifugation, using a spectrophotometer for microplates, the OD (optical density) of each supernatant was read at the wavelengths 415/630 nm. Then, the complement values were calculated and used for graphic representation. The results are shown in Fig. 20. From these results, it was confirmed that sCR1 inhibited the membrane attack complex (MAC) formation owing to the complement inhibiting effect of sCR1 in the classical pathway, namely via restricted cleavage of C3b and C4b. In the case of about 20 μg/ml-NHS, it has a complement inhibiting effect of about 50% or more in terms of CH50.

ii) ACH50 is an abbreviation for alternative complement hemolysis 50 and indicates the collective complement activity via the alternative pathway of complement activation. (It is also called AP-CH50 or AH50). In the alternative pathway of the complement system, C3, C5, C6, C7, C8 and C9 are activated in that order through the intermediary of C3, B factor, D factor and P. Antibodies, C1, C4 and C2, which participate in the classical pathway, are not involved in the alternative pathway. Rabbit erythrocytes, for instance, are known as an activating substance. Generally, the ACH50 is measured using, as an indicator, the membrane attack complex forming ability with rabbit erythrocytes (Er; guinea pig erythrocytes are also used, hence Er = erythrocyte) as the target. Thus, the group of factors in the alternative pathway in the sample are activated by Ers and the C5b-9 complex is formed on the Er membrane, and Ers are lyzed. For the activation of the alternative pathway, $Mg^{2+}$ is required (in the step of B factor activation) but $Ca^{2+}$ is not required. On the other hand, when human serum is reacted with Ers, hemolysis via the classical pathway also takes place since natural antibodies against Er are present in human serum.

Therefore, for causing hemolysis via the alternative pathway alone, not via the classical pathway, a reaction mixture containing $Mg^{2+}$ but containing no $Ca^{2+}$ is required. Therefore, EGTA, which is a chelating agent specific to $Ca^{2+}$, is used. This ACH50 measuring method, like the CH50 determination, positive detection is obtained only when all of C3, B, D, P and C5-C9 are present, and the measurement is carried out by the 50% hemolysis method and the results are expressed using ACH50/ml as a unit. The experiments were carried out according to the protocol for the microplate method (shigeharu Nagasawa: Seitai to Men-eki Bogyo I, published 1997). First 5VB was prepared and, then, GVB (gelatin 1.0 g, 5VB 100 ml, dextrose (anhydrous) 1 g, 0.15 M Ca and 1 M Mg solution 1 ml, total amount 1,000 ml) and an EGTA-Mg stock solution (1 M EGTA and 0.02 M Mg solution) were prepared. Then, EGTA·GGVB($Mg^{2+}$) (gelatin 1.0 g, 5VB 80 ml, dextrose (anhydrous) 25 g, EGTA-Mg stock 100 ml. total amount 1,000 ml), 0.9% NaCl, sCR1, and NHS were prepared. Then, $5.0 \times 10^7$ cells/ml of Ers and 1-120 µg/ml-NHS (ml) preparations (prepared by preparing a dilution series of sCR1, followed by 10-fold dilution with NHS) were prepared. EGTA-GGVB($Mg^{2+}$) and distilled water were applied to a 96-well plate except for the uppermost row, and sCR1-NHS solutions prepared in the same manner by preparing a dilution series followed by further 10-fold dilution with GVB were applied in 75-µl portions to the vacant uppermost row. Using a diluter, a 50-µl portion of the contents in each uppermost row well was transferred to the next row well therebelow and this type of transfer was repeated toward each lowermost row well to thereby produce a continuous series of 25-µl 3/2-fold dilutions. Then, 25 µl of the Er preparation prepared was added to each well, and the reaction was allowed to proceed at 37°C for 60 minutes.

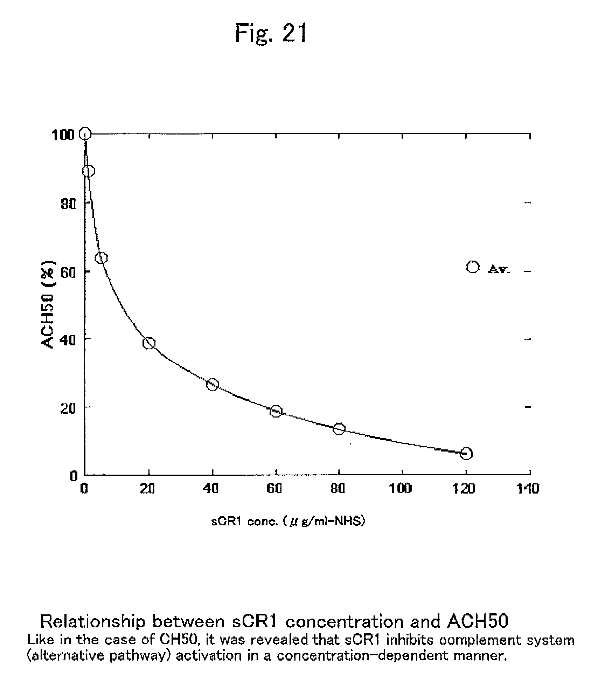

After the 60 minutes, 50 µl of EGTA-GGVB($Mg^{2+}$) was added to each well, and the mixture was centrifuged at 1,600-2,000 x g for 10 minutes. After centrifugation, the OD of each supernatant was read at the wavelengths 415/630 nm using a spectrophotometer for microplates. Then, the complement values were calculated and used for graphic representation. The results are shown in Fig. 21. From these results, it was confirmed that sCR1 inhibited the membrane attack complex (MAC) formation owing to the complement inhibiting effect of sCR1 in the alternative pathway, namely via restricted cleavage of C3b. In the case of about 20 µg/ml-NHS, it has a complement inhibiting effect of about 50% or more in terms of CH50.

iii) Quantitative determinations of the complement components iC3b and SC5b-9 by ELISA

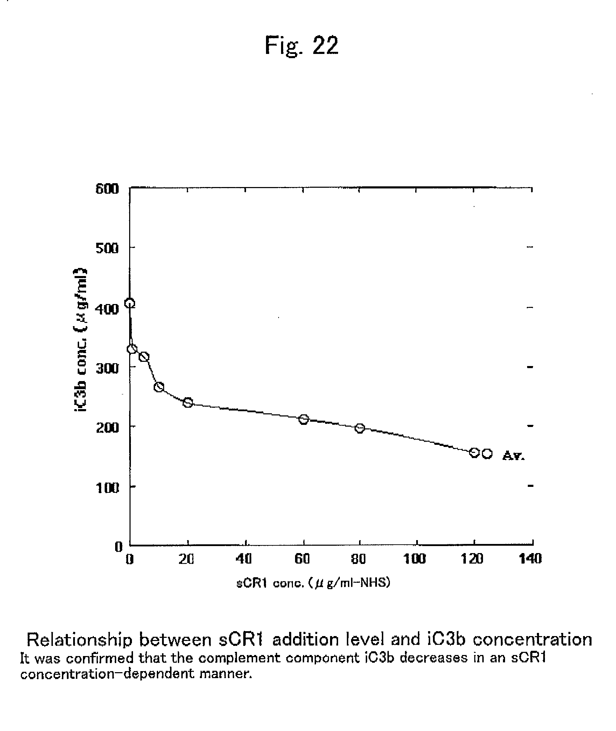

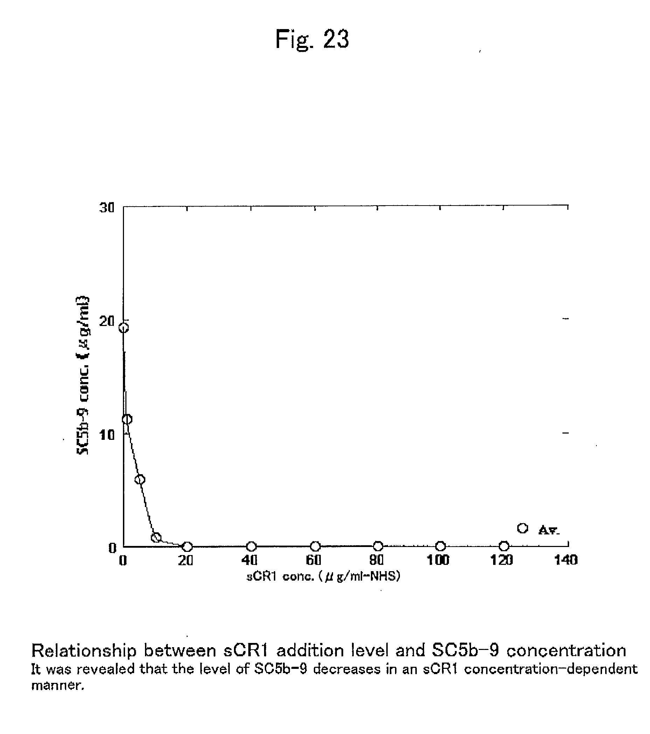

As an experiment accompanying the CH50 measurements, complement detection was carried out by ELISA. The detection kit (QUIDEL) is designed for the sandwich technique using anti-iC3b and SC5b-9 antibodies and makes it possible to quantitatively determine the level of MAC formation and the level of iC3b formation. Two kinds of supernatants (uppermost row and third row) after the reaction for CH50 determination were used, and how these complement components present in NHS changed upon actual addition of sCR1 was checked. The experiment procedure was as follows. First, sCR1 samples were prepared. (Using NHS in the original form, 200-fold and 315-fold dilutions were prepared.) Then, primary antibody-coated 96-well plates were taken out of the kit, and 300 µl of the diluted wash solution was added to each well, followed by 1 minute of standing. The wash solution (hereinafter abbreviated as w. s.) added to each well was sucked off. This washing procedure was repeated three times. The samples and so forth were distributed in 100-µl portions into the wells at predetermined places (on this occasion, NHS was added after 200-fold dilution, and SC5b-9 controls H and L were added after 25-fold dilution). After addition, the iC3b plate was incubated for 30 minutes, and the SC5b-9 plate for 60 minutes, at room temperature. After incubation, the solution in each well was sucked off, about 300 µl of w.s. was added and, after 1 minute of standing, the w.s. was sucked off. This washing procedure was repeated 5 times in all. (During this operation, the second antibody, namely HRP-conjugate, was prepared.) The iC3b or SC5b-9 conjugate Ab was distributed in 50-µl portions into the wells, and the iC3b plate was incubated for 30 minutes, and the SC5b-9 plate for 60 minutes, at room temperature. After incubation, the solution in each well was sucked off, 300 µl of w.s. was added and, after 1 minute of standing, the w.s. was sucked off. This washing procedure was repeated 5 times in all. (During this operation, the substrate was 21-fold diluted; 550 µl Sub.: 11 ml S. Diluent). The diluted substrate was distributed in 100-µl portions into the wells and, after wrapping with an aluminum foil, the plates were incubated at room temperature (shielded against light) . At the proper time for color development, the reaction was stopped by adding the stop solution. Using a microplate reader set and adjusted at the wavelength 405 nm, and absorbance measurements were carried out. A calibration curve was constructed from the values read for solutions for calibration curve construction. Then, the iC3b and SC5b-9 concentrations of the respective samples were determined using the calibration curve, and used for graphic representation (Fig. 22, Fig. 23). The following equations were used as the calibration curve equations.

iC3b protein concentration = (O.D. value obtained - 219.42)

x dilution factor/349.45

SC5b-9 protein concentration = (O.D. value obtained -

198.93) x dilution factor/2260.2

As a result, as for iC3b, the concentration thereof decreased with the increasing sCR1 concentration, as can be read from the graph (Fig. 22). Thus, the total amount of C3b decreased, and complement activation was suppressed. In the alternative pathway, restricted cleavage starts with C3 and C3 is converted to C3b by autocatalysis (C3bBb, for instance, being an autocatalyst). However, C3b, which is the basis thereof, is cleaved into iC3b by sCR1, and C3bBb, which catalyzes the restricted cleavage of C3 to C3b, is no more present, so that the total amount of C3b decreased and the cleavage of iC3b decreases. (Namely, most of C3 remains uncleaved.) For these reasons, the results such as shown in Fig. 22 were obtained in this example. When it is considered that the original concentration of iC3b contained in serum is 400-500 μg/ml, it was confirmed, based on the results obtained, that when the sCR1 concentration is 120 μg/ml-NHS, the reduction in iC3b level amounts to about half or more.

As for SC5b-9 (soluble C5b-9), it can be read from the graph that when the sCR1 concentration is increased, the level of formation of SC5b-9 decreases (Fig. 23). The results obtained indicate that when the sCR1 concentration is 20 μg/ml or above, almost no SC5b-9 formation can be observed. This shows a similarity to the CH50 and ACH50 data, and it can be considered that the activity of sCR1 used is sufficient to almost totally inhibit complement activation at a concentration of about 20 μg/ml-NHS.

iv) CH50 and ACH50 measurements with Aglyco-sCR1

Like in the case of sCR1, Aglyco-sCR1 was also assayed for its complement activation inhibiting effects with respect to CH50 and ACH50, respectively. Like in the CH50 and ACH50 assays, the following samples were prepared. (Using the sample obtained, they were prepared according to the following formulations.)

(1) A - sCR1:NHS = 200 (μl):200 (μl) = 1:1
(2) 0.9% saline:NHS = 200 (μl):200 (μl) = 1:1
(3) NHS alone

In the case of CH50
Solution (1) :GVB = 100 (μl):400 (μl) = 1:4 (5-fold dilution)
Solution (2) :GVB = 100 (μl):400 (μl) = 1:4 (5-fold dilution)
Solution (3) :GVB = 100 (μl):400 (μl) = 1:4 (5-fold dilution)
In the case of ACH50
Solution (1):EGTA-Mg$^{2+}$-GGVB = 200 (μl):200 (μl) = 1:1 (2-fold dilution)
Solution (2):EGTA-Mg$^{2+}$-GGVB = 200 (μl):200 (μl) = 1:1 (2-fold dilution)
Solution (3):EGTA-Mg$^{2+}$-GGVB = 200 (μl):200 (μl) = 1:1 (2-fold dilution)

The CH50 and ACH50 activities were measured following the same experimental procedures as mentioned above. As a result, it was confirmed that Aglyco-sCR1 can inhibit complement activation in terms of CH50 and ACH50 although its activities are lower as compared with native sCR1 on the same concentration levels.

v) Observation in a cytotoxic experiment using sCR1

In an experiment closer to the *in vivo* condition (cells perform intercellular signal transduction, so that when the complement system functions, cells help with one another and cell restoration results), it was checked, by actually handling adhesive cells, the extent of inhibition of necrosis by complement activation.

The cells used herein were of a mouse cranial bone-derived stroma cell line (PA-6). Their behavior was observed using inactivated NHS, NHS, and sCR1 after concentration adjustment. The experimental procedure was as follows. First, cells of the mouse cranial bone-derived stroma cell line (PA-6) were sowed onto a 24-well plate in advance, and allowed to proliferate until confluency. After a confluent state was obtained, two washing operations were carried out with PBS, and α-MEM was added. Then, mixtures of inactivated NHS (heated inactivated pooled NHS: 56°C, 40 min) or NHS, and the sCR1 sample after concentration adjustment with PBS (sCR1:NHS or H-NHS = 1:9;

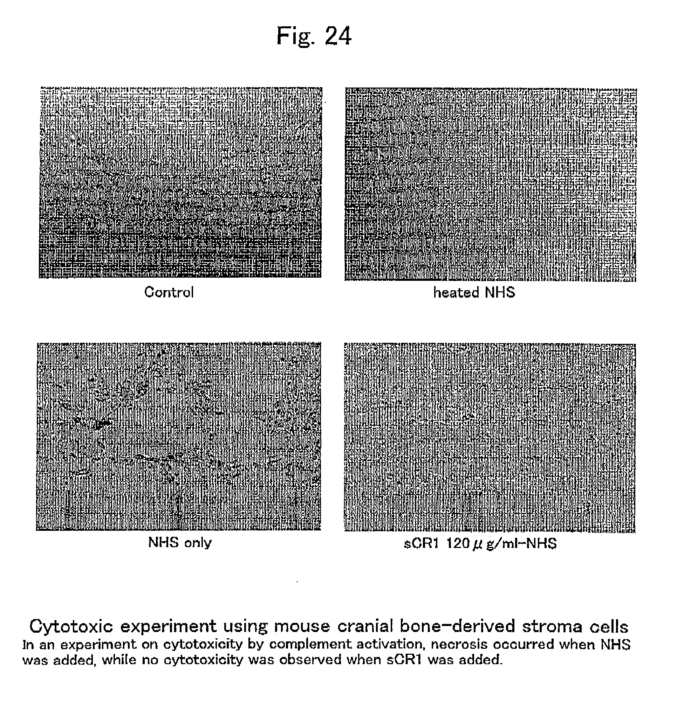

sCR1 10-fold diluted with NHS) were added in 100-μl portions and, after the lapse of 1 hour, the state in each well was observed. The results are shown in Fig. 24. From these results, it was confirmed that when NHS alone was used, the complement system was activated and cells peeled off from the dish. For the inactivated sample, it was confirmed that the complement system was completely inactivated. For the sample containing sCR1, it was confirmed that the complement inhibiting effect manifested itself from a concentration of 20 μg/ml-NHS. As described above in Example 5, (2), i) and 2), the complement inhibiting effect of sCR1 could be confirmed at considerably lower doses thereof as compared with the CH50 and ACH50 measurements when cell restoration was taken into consideration.

Example 6 Confirmation of an *in vivo* activity of sCR1

The samples of sCR1 used were purified sCR1 and Aglyco-sCR1 obtained by HPLC gel filtration column chromatography in Example 2 (2).

Confirmation of an activity of sCR1 using model rats

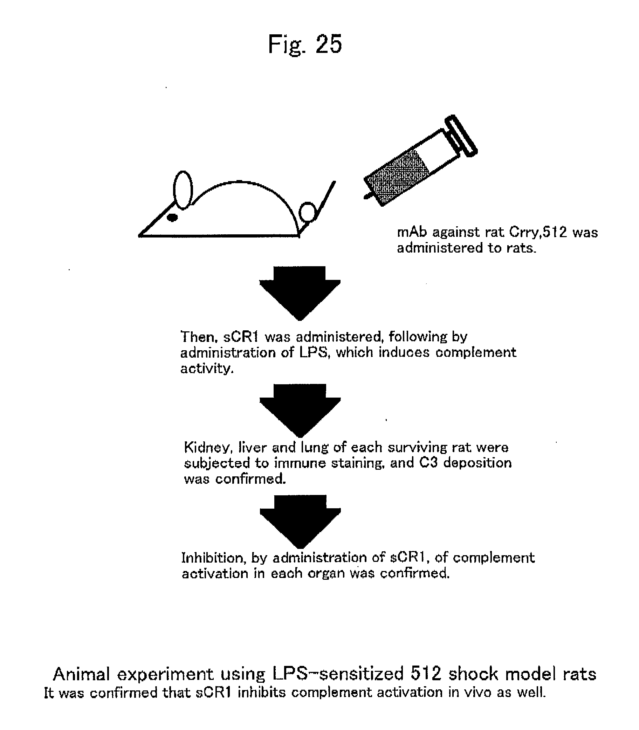

In this experiment, an in vivo activity of sCR1 was confirmed using model rats. The experimental procedure is schematically shown in Fig. 25. The procedure was as follows. First, anti-Crry antibody capable of inhibiting membrane receptors (e.g. CR1) on the cell membrane was administered to rats to thereby create a state in which the complement regulating protein could not function (nude rats showing no immune system function). Then, sCR1 was administered to the rats at a dose of 20 mg/kg. After sCR1 administration, lipoplysaccharide (LPS), a substance capable of activating the complement system, was administered. For each rat surviving thereafter, various organs were subjected to immune staining to see whether the deposition of C3 had occurred or not. No C3 deposition was found in lung, liver, and kidney. It was thus confirmed that the complement system had been inhibited. For Aglyco-sCR1, too, results were obtained indicating complement inhibition although the complement inhibiting ability was weaker as compared with intact sCR1.

In accordance with the invention, means for obtaining solubilized complement receptor type 1 (sCR1) with a very high purity has been developed and, as a result, solubilized complement receptor type 1 (sCR1) could be successfully crystallized for the first time. Therefore, in accordance with the invention, the product can be used as a complement activity inhibitor which is pure and shows reduced side effects and the crystals obtained make it possible to analyze the three-dimensional structure thereof using X-ray crystal structure analyzing techniques. The invention further may form a valuable contribution to the investigations concerning the crystallization of similar solubilized membrane proteins and the three-dimensional structure analysis thereof. In addition, the high-purity sCR1 production process with high efficiency developed for enabling crystallization for the purpose of structure analysis as intended by the invention makes it possible to stably supply high-quality sCR1 which can maintain its physiological activities at high levels. Thus, the invention can widely contribute to the medical field and to the field of new drug development.

**Claims**

1. A crystal of the soluble-form complement receptor type 1 (sCR1) in crystal form.

2. A crystal of the soluble-form complement receptor type 1 (sCR1) in crystal form according to Claim 1, obtained by crystallization of the soluble-form complement receptor type 1 purified by a process comprising the following steps a and b:

   a: propagating animal cells introduced with a gene for soluble-form complement receptor type 1 (sCR1) in a serum-containing medium and then cultivating the cells in a serum-free medium for causing them to produce soluble-form complement receptor type 1 (sCR1); and
   b: purifying the produced soluble-form complement receptor type 1 (sCR1) by the successive use of an affinity column chromatography and a size exclusion column chromatography.

3. A crystal of the soluble-form complement receptor type 1 (sCR1) according to Claim 1 or 2 which contains no transmembrane domain.

4. A crystal of the soluble-form complement receptor type 1 (sCR1) according to any of Claims 1 to 3 which has no glyco-chain.

5. A crystal of the soluble-form complement receptor type 1 (sCR1) according to any of Claims 1 to 4, wherein the purification step(s) a and/or b are(is) carried out in the presence of a surfactant.

6. A crystal of soluble-form complement receptor type 1 (sCR1) according to any of Claims 1 to 5, wherein the crystallization means comprises a combination of a precipitation procedure using a protein precipitating agent and a vapor diffusion method

7. A complement activity inhibitor which comprises a crystal of the soluble-form complement receptor type 1 (sCR1) according to any of Claims 1 to 6.

8. A method of producing a crystal of soluble-form complement receptor type 1 (sCR1) which comprises crystallizing the soluble-form complement receptor type 1 (sCR1) obtained by a process comprising the following steps a and b:

   a: propagating animal cells introduced with a gene for the soluble-form complement receptor type 1 (sCR1) in a serum-containing medium and then cultivating the cells in a serum-free medium for causing them to produce the soluble-form complement receptor type 1 (sCR1); and
   b: purifying the produced soluble-form complement receptor type 1 (sCR1) by the successive use of an affinity column chromatography and a size exclusion column chromatography.

9. A method of producing a crystal of the soluble-form complement receptor type 1 (sCR1) according to Claim 8, wherein the gene for the soluble-form complement receptor type 1 (sCR1) is one resulting from mutation or deletion in the transmembrane domain-encoding region.

10. A method of producing a crystal of soluble-form complement receptor type 1 (sCR1) according to Claim 9, wherein the soluble-form complement receptor type 1 (sCR1) is excreted extracellularly.

11. A method of producing a crystal of soluble-form complement receptor type 1 (sCR1) according to any of Claims 8 to 10, wherein the cultivation step(s) a and/or b are(is) carried out in the presence of tunicamycin.

12. A method of producing a soluble-form complement receptor type 1 (sCR1) in crystal form according to any of Claims 8 to 11, wherein the purification step(s) a and/or b are(is) carried out in a state wherein the soluble-form complement receptor type 1 (sCR1) is dispersed by means of a surfactant.

13. A method of producing a crystal of soluble-form complement receptor type 1 (sCR1 according to any of Claims 8 to 12, wherein the crystallization means comprises a combination of a precipitation procedure using a protein precipitating agent and a vapor diffusion method.

14. A two-step cell culture method comprising propagating animal cells introduced with a gene for soluble-form complement receptor type 1 (sCR1) in a serum-containing medium, followed by cultivation in a serum-free medium.

15. A two-step culture method according to Claim 14, wherein the soluble-form complement receptor type 1 (sCR1) gene is one resulting from mutation or deletion in the transmembrane domain-encoding region.

16. A two-step culture method according to Claim 15, wherein the animal cells introduced with the soluble-form complement receptor type 1 (sCR1) gene extracellularly secrete the soluble-form complement receptor type 1 (sCR1).

17. A two-step culture method according to any of Claims 14 to 16, wherein the cultivation step(s) is (are) carried out in the presence of tunicamycin.

18. A two-step process for purifying a soluble-form complement receptor type 1 (sCR1), which comprises purifying the soluble-form complement receptor type 1-containing animal cell culture medium treated or untreated, obtained by the two-step culture method according to any of Claims 14 to 17, by using an affinity column chromatography and a size exclusion column chromatography successively.

19. A two-step purification process according to Claim 18, wherein the animal cell culture supernatant containing the soluble-form complement receptor type 1 (sCR1) is directly applied to an affinity column chromatography.

20. A two-step purification process according to Claim 18 or 19, wherein the soluble-form complement receptor type 1 (sCR1) is subjected, in a dispersed state by a surfactant, to purification by using an affinity column chromatography and/or size exclusion column chromatography.

21. A complement activity inhibitor wherein the soluble-form complement receptor type 1 (sCR1) obtained by the two-step purification process according to any of Claims 18 to 20 is used.

# Fig. 1

— Complement system —

**Complement system and sCR1**

The complement system is constituted of the classical pathway and alternative pathway. The complement system is activated by sequential limited cleavages of complement components. sCR1 cleaves the intermediate complement components C3b and C4b and converts them to inactive forms.

# Fig. 2

Scheme for experiment

Two-step cell culture

sCR1-producing cells
(CHO/sCR1)

Serum-free culture

Proliferation culture

Proliferation medium (+ MTX)
+ FBS serum

Medium exchange at 100% confluency

Substance production
culture

Serum-free medium
+ MTX

Medium recovery

Two-step column
purification

First step

Affinity column chromatography

Second step

Size exclusion chromatography

Confirmation after purificatior
Western blotting
Activity checking

Function analysis

Crystallization

## Fig. 3

Two-step cell culture

After seeding cells

0-
12hr

Proliferation coluture

α-MEM +10%FBS
+MTX

24-
48hr

Exchange for serum-free mediun
(ASF) after conlfuency

▼ ASF104+ MTX

36hr

Substance production culture

Further exchange for
serum-free medium after
medium recovery

72hr

Medium recovery

# Fig. 4

Comparison between conventional culture
method and novel two-step culture process

1. Marker

2. Conventional culture method

3. Novel two-step culture process
(sCR1 is produced in serum-free medium and the
medium is recovered)

sCR1 was produced by each method and the medium was recovered.
Shown above are the results of SDS polyacrylamide electrophoresis
(7.5% gel) of the media, followed by silver staining for detection.

Fig. 5

# Fig. 6

## Two-step cell culture using tunicamycin

Cell culture

$\alpha$-MEM 10%FBS
+MTX

Medium exchange
after confluency

Tunicamycin treatment
(concentration 6 $\mu$g/ml)

$\alpha$-MEM 10%FBS
+MTX

Medium exchange
after 24 hours

ASF104+MTX

after 12 hours

ASF104+MTX

after 36 hours

ASF104+MTX

after 48 hours

It was found optimal to continue cell invasion for 24 hours and recover the
medium after 36 hours, since cells are disintegrated by tunicamycin.

# Fig. 7

Purification of sCR1 on a heparin column

Elution conditions
20mM Tris-Cl (pH 7.4)
0.45% NaCl  0.05%  CHAPS

sCR1 could be column-concentrated successfully.

# Fig. 8

**Purification of sCR1 by gel filtration column chromatography**

A: A single peak was obtained on the chromatogram. B: This peak was recovered and subjected to polyacrylamide gel electrophoresis (7.5% gel), followed by CBB staining. A single band alone was obtained.

# Fig. 9

Purification of Aglyco-sCR1 on a heparin column

Elution conditions

20mM Tris-Cl (pH 7.4)
0.45% NaCl
0.05% CHAPS

Aglyco-sCR1 alone could be column-concentrated successfully.

## Fig. 10

Purification of Aglyco-sCR1 on a gel filtration column
A: A single peak was obtained on the chromatogram. B: This peak was recovered and subjected to polyacrylamide gel electrophoresis (7.5% gel), followed by CBB staining. A single band alone was obtained, like in the case of sCR1.

# Fig. 11

Sites of antigen—antibody binding of anti—sCR1 antibodies to sCR1
Sites of binding of four anti—sCR1 antibodies to sCR1. Ell binds to LHR C and D, KuN241 to LHR A and B, and J3D3 and To5 bind to LHR A, B and C.

# Fig. 12

Molecular weight (kDa)

No primary antibody    E11    KuN241    J3D3    To5

208 ▶

131 ▶
96 ▶

◀ sCR1

Primary antibody (mouse anti CR1 mAb)

E11    KuN241    J3D3    To5

Used after 500-fold dilution

Secondary antibody (sheep anti mouse IgG HRP linked mAb)

The secondary antibody was used after 1,000-fold dilution

Results of western blotting of sCR1
For all the four antibodies, specificity to sCR1 was confirmed.

# Fig. 13

Maker E11

Molecular weight Marker  E11
(kDa)

206 ▶ ·  ·  · ·          ▬▬ ◀Aglyco-
                                  sCR1

124 ▶·

83 ▶·                    ·

42 ▶·

Primary antibody (mouse anti CR1 mAb)
E11  Used after 500-fold dilution
Secondary antibody (sheep anti mouse IgG
                              HRP linked mAb)
The secondary antibody was used after 1,000-fold dilution

Results of western blotting of Aglyco-sCR1
With Aglyco-sCR1 as well, antibody specificity was confirmed.

# Fig. 14

24-Well dish

Search for crystallization conditions

Precipitating agent solution

+

Protein solution (sCR1 or Aglyco-sCR1)

Screening with respect to precipitating agent, pH, concentration, temperature, etc.

Precipitating agent solution

Sitting drop method

Optimum crystallization conditions

## Method of producing crystals by the sitting drop vapor diffusion method
Optimum crystallization conditions were found out by the sitting drop vapor diffusion method.

Fig. 15

Crystals of sCR1

Barrel-shaped particles are sCR1 crystals.

# Fig. 16

sCR1 sample after gel filtration

## Electron microscopic observation of sCR1
Electron microscopy gave an image of monodispersed sCR1.

# Fig. 17

Sample after gel filtration

Enlargement of oligomer-forming Aglyco-sCR1

Electron microscopic observation of Aglyco-sCR1
Electron microscopy gave an image of oligomer-forming Aglyco-sCR1.

# Fig. 18

Confirmation of an activity of sCR1 in terms of cofactor activity

A: Schematic representation of restricted cleavage of C3b by sCR1 and factor I (cofactor activity).

B: Upon cofactor assaying with addition of sCR1, a chain fragments of C3b were obtained.

# Fig. 19

C3b (reduced)
+
factor I
+
Aglyco-sCR1

C3b (reduced)

◀ Aglyco-
   sCR1

◀ α chain ▶

◀ β chain ▶                    ◀ α chain fragment

                               ◀ α chain fragment

Confirmation of an activity of Aglyco-sCR1 in terms of cofactor activity
Like with sCR1, it was confirmed that Aglyco-sCR1, together with factor I, cleaves C3b
in a restricted manner.

# Fig. 20

Relationship between sCR1 concentration and CH50
It was confirmed that sCR1 inhibits complement system activation in
a concentration-dependent manner.

# Fig. 21

**Relationship between sCR1 concentration and ACH50**
Like in the case of CH50, it was revealed that sCR1 inhibits complement system
(alternative pathway) activation in a concentration-dependent manner.

# Fig. 22

Relationship between sCR1 addition level and iC3b concentration
It was confirmed that the complement component iC3b decreases in an sCR1
concentration-dependent manner.

## Fig. 23

Relationship between sCR1 addition level and SC5b-9 concentration
It was revealed that the level of SC5b-9 decreases in an sCR1 concentration-dependent manner.

# Fig. 24

Control

heated NHS

NHS only

sCR1 120μg/ml-NHS

Cytotoxic experiment using mouse cranial bone-derived stroma cells
In an experiment on cytotoxicity by complement activation, necrosis occurred when NHS was added, while no cytotoxicity was observed when sCR1 was added.

# Fig. 25

mAb against rat Crry,512 was administered to rats.

Then, sCR1 was administered, following by administration of LPS, which induces complement activity.

Kidney, liver and lung of each surviving rat were subjected to immune staining, and C3 deposition was confirmed.

Inhibition, by administration of sCR1, of complement activation in each organ was confirmed.

Animal experiment using LPS-sensitized 512 shock model rats
It was confirmed that sCR1 inhibits complement activation in vivo as well.